(19) Europäisches Patentamt
European Patent Office
Office européen des brevets

(11) **EP 2 476 440 B1**

(12) **EUROPEAN PATENT SPECIFICATION**

(45) Date of publication and mention of the grant of the patent:
**02.08.2017 Bulletin 2017/31**

(21) Application number: **10768504.2**

(22) Date of filing: **10.09.2010**

(51) Int Cl.:
***A61K 47/64*** *(2017.01)*
***A61K 38/16*** *(2006.01)*
***A61K 38/39*** *(2006.01)*
***C07K 14/025*** *(2006.01)*
***C07K 14/78*** *(2006.01)*
***A61P 31/12*** *(2006.01)*
***A61P 35/00*** *(2006.01)*

(86) International application number:
**PCT/ES2010/070590**

(87) International publication number:
**WO 2011/029980 (17.03.2011 Gazette 2011/11)**

# (54) THERAPEUTIC COMPOSITIONS FOR THE TREATMENT OF HPV-INDUCED DISEASES

THERAPEUTISCHE ZUSAMMENSETZUNGEN ZUR BEHANDLUNG HPV-INDUZIERTER ERKRANKUNGEN

COMPOSITIONS THÉRAPEUTIQUES POUR LE TRAITEMENT DE MALADIES PROVOQUÉES PAR LE PVH

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **11.09.2009 ES 200901847**

(43) Date of publication of application:
**18.07.2012 Bulletin 2012/29**

(73) Proprietor: **Proyecto de Biomedicina Cima, S.L.**
**31008 Pamplona - Navarra (ES)**

(72) Inventors:
- **BERRAONDO LÓPEZ, Pedro**
**E-31008 Pamplona - Navarra (ES)**
- **LASARTE SAGASTIBELZA, Juan José**
**E-31008 Pamplona - Navarra (ES)**
- **MANSILLA PUERTA, Cristina**
**E-31008 Pamplona - Navarra (ES)**
- **PRIETO VALTUEÑA, Jesús María**
**E-31008 Pamplona - Navarra (ES)**
- **SAROBE UGARRIZA, Pablo**
**E-31008 Pamplona - Navarra (ES)**

(74) Representative: **Hoffmann Eitle**
**Patent- und Rechtsanwälte PartmbB**
**Arabellastraße 30**
**81925 München (DE)**

(56) References cited:
**EP-A1- 1 576 967**
**EP-A2- 1 913 954**
**WO-A2-2004/069204**

- **MANSILLA C ET AL: "Immunization against hepatitis C virus with a fusion protein containing the extra domain A from fibronectin and the hepatitis C virus NS3 protein", JOURNAL OF HEPATOLOGY, MUNKSGAARD INTERNATIONAL PUBLISHERS, COPENHAGEN, DK, vol. 51, no. 3, 1 September 2009 (2009-09-01), pages 520-527, XP026541557, ISSN: 0168-8278, DOI: DOI:10.1016/J.JHEP.2009.06.005 [retrieved on 2009-06-23]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 476 440 B1

LISTA DE SECUENCIAS

<110> PROYECTO DE BIOMEDICINA CIMA, S.L.

<120> COMPOSICIONES TERAPÉUTICAS PARA EL TRATAMIENTO DE ENFERMEDADES CAUSADAS POR HPV

<130> P4869PC00

<150> P200901847
<151> 2009-09-11

<160> 72

<170> PatentIn version 3.5

<210> 1
<211> 91
<212> PRT
<213> Homo sapiens

<400> 1

```
Asn Ile Asp Arg Pro Lys Gly Leu Ala Phe Thr Asp Val Asp Val Asp
1               5                   10                  15

Ser Ile Lys Ile Ala Trp Glu Ser Pro Gln Gly Gln Val Ser Arg Tyr
            20                  25                  30

Arg Val Thr Tyr Ser Ser Pro Glu Asp Gly Ile His Glu Leu Phe Pro
        35                  40                  45

Ala Pro Asp Gly Glu Glu Asp Thr Ala Glu Leu Gln Gly Leu Arg Pro
    50                  55                  60

Gly Ser Glu Tyr Thr Val Ser Val Val Ala Leu His Asp Asp Met Glu
65                  70                  75                  80

Ser Gln Pro Leu Ile Gly Thr Gln Ser Thr Ala
                85                  90
```

<210> 2
<211> 15
<212> PRT
<213> Artificial Sequence

<220>
<223> Péptido antigénico derivado de E7 HPV

<400> 2

```
His Glu Tyr Met Leu Asp Leu Gln Pro Glu Thr Thr Asp Leu Tyr
1               5                   10                  15
```

<210> 3
<211> 15
<212> PRT
<213> Artificial Sequence

<220>
<223> Péptido antigénico derivado de E7 HPV

<400> 3

```
Thr Leu Arg Leu Cys Val Gln Ser Thr His Val Asp Ile Arg Thr
1               5                   10                  15
```

<210> 4
<211> 15
<212> PRT
<213> Artificial Sequence

<220>
<223> Péptido antigénico derivado de E7 HPV

<400> 4

```
Ile Arg Thr Leu Glu Asp Leu Leu Met Gly Thr Leu Gly Ile Val
1               5                   10                  15
```

<210> 5
<211> 15
<212> PRT
<213> Artificial Sequence

<220>
<223> Péptido antigénico derivado de E7 HPV

<400> 5

```
Leu Glu Asp Leu Leu Met Gly Thr Leu Gly Ile Val Cys Pro Ile
1               5                   10                  15
```

<210> 6
<211> 15
<212> PRT
<213> Artificial Sequence

<220>
<223> Péptido antigénico derivado de E7 HPV

<400> 6

```
Asp Leu Leu Met Gly Thr Leu Gly Ile Val Cys Pro Ile Cys Ser
1               5                   10                  15
```

<210> 7
<211> 15
<212> PRT

<213> Artificial Sequence

<220>
<223> Péptido antigénico derivado de E7 HPV

<400> 7

```
Lys Ala Thr Leu Gln Asp Ile Val Leu His Leu Glu Pro Gln Asn
1               5                   10                  15
```

<210> 8
<211> 15
<212> PRT
<213> Artificial Sequence

<220>
<223> Péptido antigénico derivado de E7 HPV

<400> 8

```
Ile Asp Gly Val Asn His Gln His Leu Pro Ala Arg Arg Ala Glu
1               5                   10                  15
```

<210> 9
<211> 15
<212> PRT
<213> Artificial Sequence

<220>
<223> Péptido antigénico derivado de E7 HPV

<400> 9

```
Leu Arg Ala Phe Gln Gln Leu Phe Leu Asn Thr Leu Ser Phe Val
1               5                   10                  15
```

<210> 10
<211> 15
<212> PRT
<213> Artificial Sequence

<220>
<223> Péptido antigénico derivado de E7 HPV

<400> 10

```
Phe Gln Gln Leu Phe Leu Asn Thr Leu Ser Phe Val Cys Pro Trp
1               5                   10                  15
```

<210> 11
<211> 15
<212> PRT
<213> Artificial Sequence

<220>
<223> Péptido antigénico derivado de E7 HPV

<400> 11

Gln Asp Tyr Val Leu Asp Leu Gln Pro Glu Ala Thr Asp Leu His
1 5 10 15

<210> 12
<211> 15
<212> PRT
<213> Artificial Sequence

<220>
<223> Péptido antigénico derivado de E7 HPV

<400> 12

Asp Ile Arg Ile Leu Gln Glu Leu Leu Met Gly Ser Phe Gly Ile
1 5 10 15

<210> 13
<211> 15
<212> PRT
<213> Artificial Sequence

<220>
<223> Péptido antigénico derivado de E7 HPV

<400> 13

Ile Arg Ile Leu Gln Glu Leu Leu Met Gly Ser Phe Gly Ile Val
1 5 10 15

<210> 14
<211> 15
<212> PRT
<213> Artificial Sequence

<220>
<223> Péptido antigénico derivado de E7 HPV

<400> 14

Glu Leu Leu Met Gly Ser Phe Gly Ile Val Cys Pro Asn Cys Ser
1 5 10 15

<210> 15
<211> 15
<212> PRT
<213> Artificial Sequence

<220>
<223> Péptido antigénico derivado de E7 HPV

<400> 15

Lys Glu Tyr Val Leu Asp Leu Tyr Pro Glu Pro Thr Asp Leu Tyr

1 5 10 15

<210> 16
<211> 15
<212> PRT
<213> Artificial Sequence

<220>
<223> Péptido antigénico derivado de E7 HPV

<400> 16

Leu Arg Thr Ile Gln Gln Leu Leu Met Gly Thr Val Asn Ile Val
1 5 10 15

<210> 17
<211> 15
<212> PRT
<213> Artificial Sequence

<220>
<223> Péptido antigénico derivado de E7 HPV

<400> 17

Ile Gln Gln Leu Leu Met Gly Thr Val Asn Ile Val Cys Pro Thr
1 5 10 15

<210> 18
<211> 15
<212> PRT
<213> Artificial Sequence

<220>
<223> Péptido antigénico derivado de E7 HPV

<400> 18

Gln Leu Leu Met Gly Thr Val Asn Ile Val Cys Pro Thr Cys Ala
1 5 10 15

<210> 19
<211> 15
<212> PRT
<213> Artificial Sequence

<220>
<223> Péptido antigénico derivado de E7 HPV

<400> 19

Ala Glu Thr Leu Gln Glu Ile Val Leu His Leu Glu Pro Gln Asn
1 5 10 15

<210> 20

<211> 15
<212> PRT
<213> Artificial Sequence

<220>
<223> Péptido antigénico derivado de E7 HPV

<400> 20

Leu Arg Thr Leu Gln Gln Leu Phe Leu Ser Thr Leu Ser Phe Val
1 5 10 15

<210> 21
<211> 15
<212> PRT
<213> Artificial Sequence

<220>
<223> Péptido antigénico derivado de E7 HPV

<400> 21

Leu Gln Gln Leu Phe Leu Ser Thr Leu Ser Phe Val Cys Pro Trp
1 5 10 15

<210> 22
<211> 15
<212> PRT
<213> Artificial Sequence

<220>
<223> Péptido antigénico derivado de E7 HPV

<400> 22

Asp Leu Arg Val Val Gln Gln Leu Leu Met Gly Ala Leu Thr Val
1 5 10 15

<210> 23
<211> 15
<212> PRT
<213> Artificial Sequence

<220>
<223> Péptido antigénico derivado de E7 HPV

<400> 23

Leu Arg Val Val Gln Gln Leu Leu Met Gly Ala Leu Thr Val Thr
1 5 10 15

<210> 24
<211> 15
<212> PRT
<213> Artificial Sequence

<220>
<223> Péptido antigénico derivado de E7 HPV

<400> 24

Val Gln Gln Leu Leu Met Gly Ala Leu Thr Val Thr Cys Pro Leu
1 5 10 15

<210> 25
<211> 15
<212> PRT
<213> Artificial Sequence

<220>
<223> Péptido antigénico derivado de E7 HPV

<400> 25

Gln Gln Leu Leu Met Gly Ala Leu Thr Val Thr Cys Pro Leu Cys
1 5 10 15

<210> 26
<211> 15
<212> PRT
<213> Artificial Sequence

<220>
<223> Péptido antigénico derivado de E7 HPV

<400> 26

Gln Leu Leu Met Gly Ala Leu Thr Val Thr Cys Pro Leu Cys Ala
1 5 10 15

<210> 27
<211> 15
<212> PRT
<213> Artificial Sequence

<220>
<223> Péptido antigénico derivado de E7 HPV

<400> 27

Lys Asp Tyr Ile Leu Asp Leu Gln Pro Glu Thr Thr Asp Leu His
1 5 10 15

<210> 28
<211> 15
<212> PRT
<213> Artificial Sequence

<220>
<223> Péptido antigénico derivado de E7 HPV

<400> 28

```
Leu Arg Thr Leu Gln Gln Met Leu Leu Gly Thr Leu Gln Val Val
1               5                   10                  15


<210>  29
<211>  15
<212>  PRT
<213>  Artificial Sequence

<220>
<223>  Péptido antigénico derivado de E7 HPV

<400>  29

Leu Gln Gln Met Leu Leu Gly Thr Leu Gln Val Val Cys Pro Gly
1               5                   10                  15


<210>  30
<211>  15
<212>  PRT
<213>  Artificial Sequence

<220>
<223>  Péptido antigénico derivado de E7 HPV

<400>  30

Gln Met Leu Leu Gly Thr Leu Gln Val Val Cys Pro Gly Cys Ala
1               5                   10                  15


<210>  31
<211>  15
<212>  PRT
<213>  Artificial Sequence

<220>
<223>  Péptido antigénico derivado de E7 HPV

<400>  31

Val Pro Thr Leu Gln Asp Val Val Leu Glu Leu Thr Pro Gln Thr
1               5                   10                  15


<210>  32
<211>  15
<212>  PRT
<213>  Artificial Sequence

<220>
<223>  Péptido antigénico derivado de E7 HPV

<400>  32

Leu Gln Asp Val Val Leu Glu Leu Thr Pro Gln Thr Glu Ile Asp
1               5                   10                  15
```

<210> 33
<211> 15
<212> PRT
<213> Artificial Sequence

<220>
<223> Péptido antigénico derivado de E7 HPV

<400> 33

Gln Asp Val Val Leu Glu Leu Thr Pro Gln Thr Glu Ile Asp Leu
1 5 10 15

<210> 34
<211> 15
<212> PRT
<213> Artificial Sequence

<220>
<223> Péptido antigénico derivado de E7 HPV

<400> 34

Cys Lys Phe Val Val Gln Leu Asp Ile Gln Ser Thr Lys Glu Asp
1 5 10 15

<210> 35
<211> 15
<212> PRT
<213> Artificial Sequence

<220>
<223> Péptido antigénico derivado de E7 HPV

<400> 35

Val Val Gln Leu Asp Ile Gln Ser Thr Lys Glu Asp Leu Arg Val
1 5 10 15

<210> 36
<211> 9
<212> PRT
<213> Artificial Sequence

<220>
<223> Péptido antigénico derivado de E7 HPV

<400> 36

Thr Leu His Glu Tyr Met Leu Asp Leu
1 5

<210> 37
<211> 9
<212> PRT

<213> Artificial Sequence

<220>
<223> Péptido antigénico derivado de E7 HPV

<400> 37

Tyr Met Leu Asp Leu Gln Pro Glu Thr
1 5

<210> 38
<211> 9
<212> PRT
<213> Artificial Sequence

<220>
<223> Péptido antigénico derivado de E7 HPV

<400> 38

Met Leu Asp Leu Gln Pro Glu Thr Thr
1 5

<210> 39
<211> 11
<212> PRT
<213> Artificial Sequence

<220>
<223> Péptido antigénico derivado de E7 HPV

<400> 39

Gln Ala Glu Pro Asp Arg Ala His Tyr Asn Ile
1 5 10

<210> 40
<211> 19
<212> PRT
<213> Artificial Sequence

<220>
<223> Péptido antigénico derivado de E7 HPV

<400> 40

Gln Ala Glu Pro Asp Arg Ala His Tyr Asn Ile Val Thr Phe Cys Cys
1 5 10 15

Lys Cys Asp

<210> 41
<211> 14
<212> PRT

<213> Artificial Sequence

<220>
<223> Péptido antigénico derivado de E7 HPV

<400> 41

```
Gln Ala Glu Pro Asp Arg Ala His Tyr Asn Ile Val Thr Phe
1               5                   10
```

<210> 42
<211> 13
<212> PRT
<213> Artificial Sequence

<220>
<223> Péptido antigénico derivado de E7 HPV

<400> 42

```
Gln Ala Glu Pro Asp Arg Ala His Tyr Asn Ile Val Thr
1               5                   10
```

<210> 43
<211> 17
<212> PRT
<213> Artificial Sequence

<220>
<223> Péptido antigénico derivado de E7 HPV

<400> 43

```
Gln Ala Glu Pro Asp Arg Ala His Tyr Asn Ile Val Thr Phe Cys Cys
1               5                   10                  15

Lys
```

<210> 44
<211> 16
<212> PRT
<213> Artificial Sequence

<220>
<223> Péptido antigénico derivado de E7 HPV

<400> 44

```
Met Leu Asp Leu Gln Pro Glu Thr Thr Asp Leu Tyr Cys Tyr Glu Gln
1               5                   10                  15
```

<210> 45
<211> 12
<212> PRT

<213> Artificial Sequence

<220>
<223> Péptido antigénico derivado de E7 HPV

<400> 45

Met Leu Asp Leu Gln Pro Glu Thr Thr Asp Leu Tyr
1 5 10

<210> 46
<211> 8
<212> PRT
<213> Artificial Sequence

<220>
<223> Péptido antigénico derivado de E7 HPV

<400> 46

Met Leu Asp Leu Gln Pro Glu Thr
1 5

<210> 47
<211> 15
<212> PRT
<213> Artificial Sequence

<220>
<223> Péptido antigénico derivado de E7 HPV

<400> 47

Arg Glu Tyr Ile Leu Asp Leu His Pro Glu Pro Thr Asp Leu Phe
1 5 10 15

<210> 48
<211> 15
<212> PRT
<213> Artificial Sequence

<220>
<223> Péptido antigénico derivado de E7 HPV

<400> 48

Thr Cys Cys Tyr Thr Cys Gly Thr Thr Val Arg Leu Cys Ile Asn
1 5 10 15

<210> 49
<211> 15
<212> PRT
<213> Artificial Sequence

<220>
<223> Péptido antigénico derivado de E7 HPV

<400> 49

```
Val Arg Thr Leu Gln Gln Leu Leu Met Gly Thr Cys Thr Ile Val
1               5                   10                  15
```

<210> 50
<211> 15
<212> PRT
<213> Artificial Sequence

<220>
<223> Péptido antigénico derivado de E7 HPV

<400> 50

```
Leu Gln Gln Leu Leu Met Gly Thr Cys Thr Ile Val Cys Pro Ser
1               5                   10                  15
```

<210> 51
<211> 30
<212> PRT
<213> Artificial Sequence

<220>
<223> péptido antigénico derivado de HPV E7 que contiene los aminoácidos 1 a 29 de E7

<400> 51

```
Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn His
            20                  25                  30
```

<210> 52
<211> 56
<212> PRT
<213> Artificial Sequence

<220>
<223> Péptido antigénico derivado de HPV E7 que contiene los aminoácidos 43 a 98 de E7

<400> 52

```
Gly Gln Ala Glu Pro Asp Arg Ala His Tyr Asn Ile Val Thr Phe Cys
1               5                   10                  15

Cys Lys Cys Asp Ser Thr Leu Arg Leu Cys Val Gln Ser Thr His Val
            20                  25                  30

Asp Ile Arg Thr Leu Glu Asp Leu Leu Met Gly Thr Leu Gly Ile Val
```

35 40 45

Cys Pro Ile Cys Ser Gln Lys Pro
50 55

<210> 53
<211> 191
<212> PRT
<213> Artificial Sequence

<220>
<223> Proteína de fusión que comprende la región EDA flanqueada por dos péptidos antigénicos de HPV E7

<400> 53

Met His Gly Asp Thr Pro Thr Leu His Glu Tyr Met Leu Asp Leu Gln
1 5 10 15

Pro Glu Thr Thr Asp Leu Tyr Cys Tyr Glu Gln Leu Asn His Met Asn
20 25 30

Ile Asp Arg Pro Lys Gly Leu Ala Phe Thr Asp Val Asp Val Asp Ser
35 40 45

Ile Lys Ile Ala Trp Glu Ser Pro Gln Gly Gln Val Ser Arg Tyr Arg
50 55 60

Val Thr Tyr Ser Ser Pro Glu Asp Gly Ile Arg Glu Leu Phe Pro Ala
65 70 75 80

Pro Asp Gly Glu Asp Asp Thr Ala Glu Leu Gln Gly Leu Arg Pro Gly
85 90 95

Ser Glu Tyr Thr Val Ser Val Val Ala Leu His Asp Asp Met Glu Ser
100 105 110

Gln Pro Leu Ile Gly Ile Gln Ser Thr Ala Ala Ala Gly Gln Ala Glu
115 120 125

Pro Asp Arg Ala His Tyr Asn Ile Val Thr Phe Cys Cys Lys Cys Asp
130 135 140

Ser Thr Leu Arg Leu Cys Val Gln Ser Thr His Val Asp Ile Arg Thr
145 150 155 160

Leu Glu Asp Leu Leu Met Gly Thr Leu Gly Ile Val Cys Pro Ile Cys

```
                165                 170                 175

Ser Gln Lys Pro Ala Ala Ala Leu Glu His His His His His His
            180                 185                 190


<210>  54
<211>  14
<212>  PRT
<213>  Artificial Sequence

<220>
<223>  Péptido enlazador/espaciador

<400>  54

Ser Gly Gly Thr Ser Gly Ser Thr Ser Gly Thr Gly Ser Thr
1               5                   10


<210>  55
<211>  15
<212>  PRT
<213>  Artificial Sequence

<220>
<223>  Péptido enlazador/espaciador

<400>  55

Ala Gly Ser Ser Thr Gly Ser Ser Thr Gly Pro Gly Ser Thr Thr
1               5                   10                  15


<210>  56
<211>  7
<212>  PRT
<213>  Artificial Sequence

<220>
<223>  Péptido enlazador/espaciador

<400>  56

Gly Gly Ser Gly Gly Ala Pro
1               5


<210>  57
<211>  8
<212>  PRT
<213>  Artificial Sequence

<220>
<223>  Péptido enlazador/espaciador

<400>  57

Gly Gly Gly Val Glu Gly Gly Gly
```

1 5

<210> 58
<211> 7
<212> PRT
<213> Artificial Sequence

<220>
<223> Péptido enlazador/espaciador

<400> 58

Gly Thr Lys Val His Met Lys
1 5

<210> 59
<211> 10
<212> PRT
<213> Artificial Sequence

<220>
<223> Péptido enlazador/espaciador

<400> 59

Pro Lys Pro Ser Thr Pro Pro Gly Ser Ser
1 5 10

<210> 60
<211> 11
<212> PRT
<213> Artificial Sequence

<220>
<223> Péptido enlazador/espaciador

<400> 60

Ala Pro Ala Glu Thr Lys Ala Glu Pro Met Thr
1 5 10

<210> 61
<211> 5
<212> PRT
<213> Artificial Sequence

<220>
<223> Secuencia diana/sitio de corte de la enteroquinasa

<400> 61

Asp Asp Asp Asp Lys
1 5

<210> 62

<211> 5
<212> PRT
<213> Artificial Sequence

<220>
<223> Secuencia diana/sitio de corte de factor Xa

<400> 62

Ile Glu Asp Gly Arg
1 5

<210> 63
<211> 6
<212> PRT
<213> Artificial Sequence

<220>
<223> Secuencia diana/sitio de corte de la trombina

<400> 63

Leu Val Pro Arg Gly Ser
1 5

<210> 64
<211> 7
<212> PRT
<213> Artificial Sequence

<220>
<223> Secuencia diana/sitio de corte de la proteasa TEV

<400> 64

Glu Asn Leu Tyr Phe Gln Gly
1 5

<210> 65
<211> 8
<212> PRT
<213> Artificial Sequence

<220>
<223> Secuencia diana/sitio de corte de la proteasa PreScission

<400> 65

Leu Glu Val Leu Phe Gln Gly Pro
1 5

<210> 66
<211> 20
<212> DNA
<213> Artificial Sequence

<220>
<223>  Motivo CpG-fosforotiato de tipo B

<400>  66
tccatgacgt tcctgacgtt
20

<210>  67
<211>  9
<212>  PRT
<213>  Artificial Sequence

<220>
<223>  Péptido sintético E7(49-57)

<400>  67

Arg Ala His Tyr Asn Ile Val Thr Phe
1               5

<210>  68
<211>  23
<212>  DNA
<213>  Artificial Sequence

<220>
<223>  Cebador UP-1

<400>  68
catatgcatg gagatacacc tac
23

<210>  69
<211>  26
<212>  DNA
<213>  Artificial Sequence

<220>
<223>  Cebador DW-1

<400>  69
gcggccgctg gtttctgaga acagat
26

<210>  70
<211>  24
<212>  DNA
<213>  Artificial Sequence

<220>
<223>  Cebador DW-2

<400>  70
catatgattt aattgctcat aaca
24

<210> 71
<211> 26
<212> DNA
<213> Artificial Sequence

<220>
<223> Cebador UP-2

<400> 71
gcggccgcag gacaagcaga accgga
26

<210> 72
<211> 185
<212> PRT
<213> Artificial Sequence

<220>
<223> Proteína de fusión sin cola de histidinas

<400> 72

```
Met His Gly Asp Thr Pro Thr Leu His Glu Tyr Met Leu Asp Leu Gln
1               5                   10                  15

Pro Glu Thr Thr Asp Leu Tyr Cys Tyr Glu Gln Leu Asn His Met Asn
            20                  25                  30

Ile Asp Arg Pro Lys Gly Leu Ala Phe Thr Asp Val Asp Val Asp Ser
        35                  40                  45

Ile Lys Ile Ala Trp Glu Ser Pro Gln Gly Gln Val Ser Arg Tyr Arg
    50                  55                  60

Val Thr Tyr Ser Ser Pro Glu Asp Gly Ile Arg Glu Leu Phe Pro Ala
65                  70                  75                  80

Pro Asp Gly Glu Asp Asp Thr Ala Glu Leu Gln Gly Leu Arg Pro Gly
                85                  90                  95

Ser Glu Tyr Thr Val Ser Val Val Ala Leu His Asp Asp Met Glu Ser
            100                 105                 110

Gln Pro Leu Ile Gly Ile Gln Ser Thr Ala Ala Ala Gly Gln Ala Glu
        115                 120                 125

Pro Asp Arg Ala His Tyr Asn Ile Val Thr Phe Cys Cys Lys Cys Asp
    130                 135                 140

Ser Thr Leu Arg Leu Cys Val Gln Ser Thr His Val Asp Ile Arg Thr
```

```
145                 150                 155                 160

Leu Glu Asp Leu Leu Met Gly Thr Leu Gly Ile Val Cys Pro Ile Cys
                165                 170                 175

Ser Gln Lys Pro Ala Ala Ala Leu Glu
            180                 185
```

## Description

Technical Field of the Invention

The invention relates to therapeutic compositions for the treatment of diseases caused by the human papilloma virus (HPV) and more specifically to compositions comprising at least one HPV E7-derived antigenic peptide.

Background of the Invention

Cervical cancer is one of the commonest cancers in women all over the world, and the fifth most frequent cancer in general, with an estimated prevalence of 1.4 million cases. There is consistent evidence that chronic genital tract infection due to various types of mucosatropic human papillomavirus causes cervical cancer.

The currently used vaccine against uterine cancer of the United States Merck laboratory, called Gardasil, can protect by 95 percent against the type 16 and 18 strains of the virus, which are in the origin of approximately 70 percent of all uterine cancer cases. It also protects against the type 6 and 11 strains, causing warts in genital organs.

The expression of the oncogenic proteins of HPV E6 and E7 is necessary for the start and the maintenance of malignant transformation and the cell immunity to E7 which have been associated with the clinical and cytological resolution of HPV-induced lesions.

The papilloma virus is a DNA virus infecting a large variety of species. Some of these viruses are associated with the development of diseases in their natural host. More than 60 types of human papillomaviruses (HPV) have been identified.

These viruses infect the human organism in multiple regions of the body, and are responsible for common warts of the skin, laryngeal papilloma, etc.

HPV genital infections are relatively frequent and the HPV types which most frequently infect the genital tract of men and women are types 6, 11, 16 and 18.

In women, HPV infects different portions of the genital tract including the cervix. Genital HPVs are a clinical problem since the infection of the anogenital region is considered to be the most frequent one of sexually transmitted diseases. HPVs cause genital infection which is presented in the following forms:

i) clinical infection, in which genital warts are presented;
ii) subclinical infection, in which the lesions are not obvious, but the viral lesions can be detected using special techniques, such as Papanicolaou cytologies; and
iii) latency, in which the single sign of infection is the presence of the HPV DNA.

On the other hand, subclinical infections are relatively common since it is estimated that 2 to 4% of Papanicolaou cytologies show evidence of HPV. The latent infections are more frequent and it is estimated that most adults have one or more types of HPV.

Uterine cervix carcinoma is a cancer common in women. Squamous cell carcinoma, which is the most commonly found (about 90% of the observed cases) and adenocarcinoma, which is a secretory cell cancer, are distinguished within this cancer. Cervical cancer has several stages. The precancerous stage is called intraepithelial neoplasia (CIN) which can evolve to invasive forms (carcinomas). Both in its precancerous form and in its invasive form, cervical cancer is one of the few cancers which can be diagnosed using a relatively reliable and inexpensive technique: the Papanicolaou test.

Evidence gathered in recent years suggests that some of the HPV types are associated with the development of cervical cancer, some of these types being etiological factors responsible for the development of cancer.

It has been postulated that HPV acts as a cervical carcinogenesis initiator and that the malignant transformation depends on the interaction with other factors. The nature of HPV-16 in particular and of the papillomaviruses in general has been studied in recent years. HPV-16 contains a double-stranded DNA genome of 7904 bp (Seedorf, K. et al., 1985, Virology 145:181-185). The capsid is 50 nm in diameter and contains 72 capsomeres (Klug, A. J., 1965, Mol. Biol. 11:403-423). Patent US 4,777,239 teaches a series of 17 synthetic peptides capable of generating antibodies against HPV-16, therefore being useful for diagnostic purposes.

HPV encodes two proteins, E6 and E7, which seem to be involved in the pathogenesis of HPV-induced abnormal cell proliferation (see Stoppler et al., 1994, Intervirology, 37:168-179). The amino acid sequences of the E6 and E7 proteins of HPV-16 were defined by Seedorf et al., (Virology, 1985, 145: 181-185).

It has been observed that the genes encoding the E6 and E7 proteins appear invariably expressed in cervical cancer tumor cells associated with HPV infection.

It is believed that the E6 and E7 proteins are an immunological target effective for tumor regression. Nevertheless, the likelihood of developing vaccines based on DNA encoding said proteins can result in the onset of irreversible cell transformation events in the cells of the patient. EP0796273 describes a composition capable of generating an

immune response in the patient without generating an unwanted cell transformation, using fusion proteins with mutated and non-mutated segments of the E6 and E7 proteins.

Préville et al., (Cancer Res., 2005, 65:641-649) describes different fusion proteins comprising adenylate cyclase (CyaA) of *Bordetella pertussis* and the complete E7 protein or different regions thereof. Adenylate cyclase is capable of binding to CD11b+ dendritic cells by interaction with the $\alpha_M\beta_2$ integrin (CD11b/CD18), therefore some of the fusion proteins are capable of triggering a specific immune response (helper T and cytotoxic T) against tumor cells expressing HPV16 E7.

Berraondo P et al., (Cancer research, 2007, 67:8847:8855) have described compositions formed by a recombinant protein which comprises CyaA and a truncated form of HPV16 lacking amino acids 30 to 42 (CyaA-E7Δ30-42), CpG associated with a cationic lipid and cyclophosphamide. This composition is capable of generating an immune response against tumors expressing E7.

However, there is still a need for additional immunogenic compositions capable of generating an immune response in the patient against tumor cells expressing HPV proteins without generating an unwanted cell transformation as well as of compositions comprising said proteins and additional components acting synergistically in the generation of the immune response such that the doses of said proteins can be reduced.

## Summary of the Invention

In a first aspect, the invention relates to a conjugate comprising:

(i) the fibronectin EDA region or a functionally equivalent variant thereof showing a degree of identity with respect to the fibronectin EDA region greater than at least 70% and
(ii) at least one HPV E7-derived antigenic peptide according to the claims,

wherein components (i) and (ii) are covalently coupled and wherein the conjugate promotes a cytotoxic response towards the antigenic peptide or peptides.

In additional aspects, the invention relates to a polynucleotide or gene construct encoding a conjugate of the invention, with a vector comprising a polynucleotide of the invention or a gene construct of the invention and with a cell containing a polynucleotide of the invention, a gene construct of the invention or a vector of the invention.

In another aspect, the invention relates to a first composition comprising, together or separately:

(i) a conjugate of the invention, a polynucleotide or gene construct of the invention, a vector of the invention, a host cell according to the invention and
(ii) a TLR ligand.

In another aspect, the invention relates to a second composition comprising, together or separately:

(i) a conjugate according to any of claims 1 to 6, a polynucleotide or gene construct according to claim 7, a vector according to claim 8, a host cell according to claim 9,
(ii) a TLR ligand and
(iii) a chemotherapeutic agent.

In another aspect, the invention relates to a pharmaceutical composition or pharmaceutical composition of the invention comprising a conjugate of the invention, a polynucleotide of the invention, a gene construct of the invention, a vector of the invention, a cell of the invention, a first or second composition of the invention and at least one pharmacologically accepted adjuvant.

In another aspect, the invention relates to a conjugate of the invention, a polynucleotide of the invention, a gene construct of the invention, a vector of the invention, a cell of the invention, a first or second composition of the invention or a pharmaceutical composition of the invention for its use in medicine.

In another aspect, the invention relates to the use of a conjugate of the invention, a polynucleotide of the invention, a gene construct of the invention, a vector of the invention, a cell of the invention, a first or second composition of the invention or a pharmaceutical composition of the invention for the manufacture of a vaccine.

In another aspect, the invention relates to the use of a conjugate of the invention, a polynucleotide of the invention, a gene construct of the invention, a vector of the invention, a cell of the invention, a first or second composition of the invention or a pharmaceutical composition of the invention for the manufacture of a medicament for the prevention and the treatment of an infection caused by the human papilloma virus and/or of cervical cancer associated with HPV infections.

In another aspect, the invention relates to an *in vitro* method or method of the invention for obtaining mature

dendritic cells presenting at least one HPV E7 antigen comprising:

(i) contacting dendritic cells with a conjugate of the invention, a polynucleotide of the invention, a gene construct of the invention, a vector of the invention, a cell of the invention, a first or second composition of the invention, in conditions suitable for the maturation of the dendritic cells to take place and
(ii) recovering the mature dendritic cells.

In another aspect, the invention relates to cells which are obtained by a method of the invention, or second cells of the invention.
In another aspect, the invention relates to second cells of the invention for their use in medicine.
In another aspect, the invention relates to the use of second cells of the invention for the manufacture of a medicament for the prevention and the treatment of an infection caused by the human papilloma virus and/or of cervical cancer associated with HPV infections.

Brief Description of the Drawings

Figure 1: Schematic representation of the recombinant EDA-HPVE7 fusion protein. The 29 first amino acids from E7 protein were placed at the N terminus of EDA, whereas amino acids 43-98 of E7 were inserted at the C terminus of EDA. The sequence of the fusion protein is shown using the single code for amino acids. Amino acids from E7 protein are shown in gray whereas the EDA amino acids are written in black. SDS-PAGE analysis of the EDA-HPVE7 recombinant protein. Five micrograms of the purified proteins were separated on a 15% SDS polyacrylamide gel and stained by Coomassie blue.
Figure 2: Activation of bone marrow-derived dendritic cell maturation by the EDAHPV-E7 fusion protein. Bone marrow-derived dendritic cells were incubated for 48 hours with EDA-HPVE7 (500 nM), with peptide E7 (49-57) (500 nM), with LPS (1 μg/ml) (positive control) or with culture medium (Control Neg). Cells were then harvested and analyzed by flow cytometry for the expression of the H-2b molecule or the maturation markers CD54 and CD86 (RFU: Relative fluorescence units).
Figure 3: EDA-HPV-E7 fusion protein induces the production of IL-12 by bone marrow-derived dendritic cells or the production of TNF-α by the human monocyte cell line THP-1. (A) Bone marrow-derived dendritic cells were incubated for 48 hours with EDA-HPVE7 (500 nM), with peptide E7(49-57)(500 nM), with LPS (1 μg/ml)(positive control) or with culture medium (negative control). 24 hours later, the culture supernatant was harvested and the IL-12 released to the supernatant was measured by ELISA. (B) THP-1 cells were incubated for 15 hours with EDA-HPVE7 (500 nM), with peptide E7(49-57)(500 nM), with LPS (1 μg/ml) or with culture medium (negative control). After culture, the supernatants were harvested and the released TNF-α was measured by ELISA.
Figure 4: *In vivo* induction of CTL by immunization with EDA-HPV-E7 protein or with the DTc/E7(49-57) peptide in PBS. (A) Mice were immunized i.v with 2 nmol EDA-HPV-E7 or with the DTc/E7(49-57) peptide. Seven days alter immunization, the mice were sacrificed and spleen cells were cultured in the presence or absence of DTc peptide for 5 days. CTL activity was measured using a conventional chromium release assay using EL4 cells radio labeled and pulsed with DTc peptide as target cells in the splenocytes cultures obtained from the mice immunized with the protein EDA-HPV-E7 (closed circles) o with the peptide DTc/E7 (49-57) (open circles) (On the graphic, the absolute values are shown, said values are calculated by subtracting the value of lysis obtained without peptide from the value obtained in the presence of peptide). (B) Measurement by ELISPOT of IFN-γ producing cells in response to the peptide E7(49-57) or of TC1 or EL-4 cells irradiated in splenocytes from mice immunized with EDA-HPV-E7 or with DTc/E7(49-57) peptide.
Figure 5: *In vivo* induction of CTL by immunization with EDA-HPV-E7 protein or peptide DTc/E7 (49-57) mixed with pI:C. Mice were immunized i.v with 2 nmol of EDA-HPV-E7 or with DTc/E7(49-57) peptide in the presence of pI:C (50μg/mice). Seven days after immunization, the mice were sacrificed and spleen cells were cultured in the presence or absence of the DTc/E7(49-57) peptide for 5 days. CTL activity was measured using a conventional chromium release assay using EL4 cells radio labeled and pulsed with the DTc peptide (target cells) (A) or irradiated TC-1 cells (B), in the spleen cell cultures obtained from mice immunized with the EDA-HPV-E7 +pI:C protein (closed circles) or with the DTc/E7(49-57)+pI:C peptide (open circles) as target cells. (C) Measurement of IFN-γ producing cells in response to the E7(49-57) peptide or to TC1 cells or EL-4 cells irradiated, in splenocytes from mice immunized with EDA-HPV-E7+pI:C or with DTc/E7(49-57)+pI:C.
Figure 6: Therapeutic efficacy of the EDA-HPVE7 fusion protein combined with poly I:C (pI:C). Mice were injected with 5 x $10^5$ TC-1 cells and 25 days later, when tumor mean diameters were approximately 8 mm, they were treated intravenously with different vaccine combinations based on EDA (A). Tumor size, presented as the average of two

perpendicular diameters (millimeters), was measured at regular intervals. The number of tumor-free mice on day 100 relative to the total number of animals included and the percent surviving on day 100 are indicated for each set of experiments. The p-value (*p<0.05) determined by the likelihood ratio test comparing tumor growth in the EDA-E7/pI:C treated group with tumor growth in the p:IC-treated group is indicated. The mice were sacrificed when the tumor diameters reached 20 mm or when necessary due to the sanitary status of the animals. (B) Survival curves from the immunized mice with the indicated immunogens are indicated (p values are indicated ((*p<0.05)). Pooled data of two independent experiments are shown.

Figure 7: Therapeutic efficacy of the EDA-HPVE7 fusion protein in a large tumor model. (A)C57BL/6 mice were injected with 5 x $10^5$ TC-1 cells and 35 days later, when tumor mean diameters were approximately 12-15 mm, the mice were left untreated or received 175 mg/kg of cyclophosphamide (CPA) specifically in a single administration. On day 36, the control mice groups were injected with PBS or 30 μg of CpG-B/DOTAP i.v. The vaccinated mice received 50 μg of EDA-E7 and 30 μg of CpG-B/DOTAP i.v. The control group treated with CpGB/DOTAP received a second dose of cyclophosphamide on day 50 and CpGB/DOTAP on day 51. The treated mice received a second dose of vaccine by injecting cyclophosphamide on day 50 and EDA-HPV-E7/CpG-B/DOTAP on day 51. (B) Tumor size, represented as the average of two perpendicular diameters (millimeters), was measured at regular intervals. The number of tumor-free mice on day 150 relative to the total number of animals included and the percent surviving on day 150 are indicated for each set of experiments. The mice were sacrificed when the tumor diametera reached 22 mm or when necessary due to the sanitary status of the animals. (C) Survival curves from the immunized mice with the indicated immunogens are indicated (p values are indicated ((*p<0.05)). Pooled data of two independent experiments are shown.

## Detailed Description of the Invention Conjugate of the invention

The authors of the present invention have observed that a recombinant protein comprising the fibronectin EDA region and a HPV-E7 E7 protein immunogenic region is capable of inducing the maturation of dendritic cells *in vitro* as well as of generating a potent antitumor activity mediated by CD8+ T cells against tumors expressing said protein. Thus, as observed in Example 1 of the present invention, contacting the previously mentioned fusion protein with bone marrow-derived dendritic cells causes the maturation of said cells as deduced from the determination of the expression levels of different dendritic cell maturation markers. Additionally, as observed in Example 2 of the present invention, the administration of said fusion protein to animal models is capable of generating an immune response mediated by cytotoxic T cells against tumor cells expressing the E7 protein.

Thus, in a first aspect, the invention relates to a conjugate (hereinafter, conjugate of the invention) comprising:

(i) the fibronectin EDA region or a functionally equivalent variant thereof and
(ii) at least one HPV E7-derived antigenic peptide, according to the claims, wherein components (i) and (ii) are covalently coupled and wherein the conjugate promotes a cytotoxic response towards the antigenic peptide or peptides.

The first element of the conjugate is the fibronectin EDA region or a functionally equivalent variant thereof showing a degree of identity with respect to the fibronectin EDA region greater than at least 70 %. The terms "EDA region" or "extra type III" are indistinctly used herein and refer to a region of the fibronectin molecule resulting from the transcription/translation of an exon of the fibronectin gene and which shows an affinity specific for the Toll-like receptors 4 (TLR4). This domain was originally described by Muro A. F. et al.; (J. Cell. Biol., 2003, 162:149-160). The EDA region may be derived from fibronectin obtained from different species such as mouse fibronectin (GenBank accession number AAD12250.1) or rat fibronectin (GenBank accession number AAB40865.1).

As used herein, "fibronectin" is understood as a multifunctional high molecular weight glycoprotein present in blood and in the extracellular matrix of tissues. Fibronectin is a dimer formed by two identical polypeptide chains bound by C-terminal disulfide bonds. Each monomer has an approximate molecular weight of 230-250 kDa. Each monomer contains three types of modules: type I, type II and type III. Each of these modules is formed by two anti-parallel β-helices.

"Functionally equivalent variant" is understood as all those peptides derived from the EDA sequence by means of modification, insertion and/or deletion of one or more amino acids, provided that the function of binding to TLR4 receptors and of activating dendritic cells is substantially maintained. Functionally equivalent variants are those showing a degree of identity with respect to the fibronectin EDA domain greater than at least 70%, at least 80%, at least 90%, at least 95%, at least 96%, at least 97%, at least 98% or at least 99%. The degree of identity between two amino acid sequences can be determined by conventional methods, for example, by means of standard sequence alignment algorithms known in the state of the art, such as, for example BLAST (Altschul S.F. et al. Basic local alignment search tool. J Mol Biol. 1990 Oct 5; 215(3):403-10). The person skilled in the art will understand that amino acid sequences referred to in this description can be chemically modified, for example, by means of chemical modifications which are physiologically relevant, such as, phosphorylations, acetylations, etc. Thus, as used herein, the expression "functionally equivalent variant" means that the polypeptide or protein in question maintains at least one of the functions of the fibronectin EDA region, preferably at least one function related to the immune response, in particular, which maintains the capacity to interact with TLR4 and to promote the maturation of dendritic cells. The capacity of the functionally equivalent variant to interact with TLR4 can be determined by means of using conventional methods known by the persons skilled in the art. For example, by way of a simple illustration, the capacity of the fibronectin EDA region variant to bind to TLR4 can be determined using co-immunoprecipitation experiments, in which the protein of interest (e.g. EDA variant) is isolated with a specific antibody and the molecules which interact with the protein (e.g. TLR4) are subsequently identified by means of a western blot. A yeast two-hybrid assay or electrophoresis assays in native conditions can also be used. The latter methodology is based on the migration of the protein complexes in polyacrylamide gels based on their molecular weight. Given that the migration is also defined by the charge, a solution containing Coomassie blue, conferring a net negative charge to the proteins without denaturing or breaking their interactions with other proteins, is used as cathode buffer. A second denaturing dimension in SDS-PAGE gels allows separating the spots and subsequently identifying the identity of the subunits forming the complex by means of mass spectrometry. Assays for determining the capacity of the functionally equivalent variants of EDA to promote the maturation of dendritic cells are known by a person skilled in the art, such as for example the assay described in Example 1 of the present application based on determining the expression levels of different mature dendritic cell markers such as IAb, CD54, CD86 and IL-12.

The person skilled in the art understands that the mutations in the nucleotide sequence encoding the EDA domain sequences which give rise to conservative substitutions of amino acids in non-critical positions for the functionality of the protein, are evolutively neutral mutations which do not affect its overall structure or its functionality.

In a preferred embodiment, the fibronectin EDA region of the conjugate of the invention corresponds to amino acids 1,631 to 1,721 of human fibronectin as shown in the UniProt database with accession number FINC HUMAN and which corresponds to the polypeptide of sequence SEQ ID NO:1.

Component (ii) of the conjugates of the invention is one or more antigenic peptides derived from the HPV E7 protein according to the claims. The E7 protein can come from different HPV serotypes. Thus, E7 proteins which can be used in the context of the present invention include, without limitation, the protein E7 of the human HPV serotype 80 (GenBank: CAA75471.1), E7 of the human HPV serotype 68 (GenBank: ACL12352.1), E7 of the human HPV serotype 11 (GenBank: ACL12343), E7 of the human HPV serotype 59 (GenBank: ACL12335), E7 of the human HPV serotype 33 (GenBank: ACL12327), E7 of the human HPV serotype 72 (GenBank: CAA63874), E7 of the human HPV serotype 16 (GenBank: ACL12311), E7 of the human HPV serotype 13 (GenBank: ABC79058), E7 of the human HPV serotype 73 (GenBank: CAA63883), E7 of the human HPV serotype 96 (GenBank: AAQ88290), transforming variant of the protein E7 of the human HPV serotype 31 (GenBank: AAR13015), E7 of the human HPV serotype 25 (GenBank: BAA09117), E7 of the human HPV serotype 21 (GenBank: BAA09116), E7 of the human HPV serotype 20 (GenBank: BAA09115), E7 of the human HPV serotype 14 (GenBank: BAA09114), E7 of the human HPV serotype 12 (GenBank: BAA09113), E7 of the human HPV serotype 27b (GenBank: BAE16264), E7 of the human HPV serotype 77 (GenBank: CAA75457), E7 of the human HPV serotype 76 (GenBank: CAA75457), E7 of the human HPV serotype 75 (GenBank: CAA75450), E7 of the human HPV serotype 2 (GenBank: NP_077117), oncogenic E7 of the human HPV serotype 38 (GenBank: CAQ16115, CAQ16114, CAQ16113, CAQ16112, CAQ16111, CAQ16110, CAQ16109, CAQ16108, CAQ16107, CAQ16106, CAQ16105, CAQ16104, CAQ16103, CAQ16102, CAQ16101, CAQ16100), E7 of the human HPV serotype 94 (GenBank: CAF05710), E7 of the human HPV serotype 95 (GenBank: CAF05703), E7 of the human HPV serotype 43 (GenBank: CAF05784), E7 of the human HPV serotype 5b (GenBank: BAA42818), E7 of the human HPV serotype 103 (GenBank: YP_656493), E7 of the human HPV serotype 101 (GenBank: AAZ39507), E7 of the human HPV serotype 103 (GenBank: AAZ39485).

As used herein, the expression "antigenic peptide" refers to a peptide molecule which comprises one or more epitopes capable of stimulating the immune system of an organism to generate a antigen-specific cell or humoral response. As a result of contacting the antigenic peptide with the suitable cells in a subject, the antigen generates a state of sensitivity or capacity for immune response in said subject such that both antibodies and immune cells obtained from said subject are capable of specifically reacting with the antigen.

As used herein, the expression "HPV E7 protein-derived antigenic peptide" means a fragment of the HPV E7 protein which is capable of stimulating the immune system of a mammal such that an immune response against said protein capable of inhibiting the growth of tumors caused by the expression of E7 or of inhibiting the proliferation of HPV is generated.

It will be observed that the antigen or the antigens can be the complete E7 protein, as well as isolated domains of said protein, peptide fragments of the E7 protein or polyepitope fusion proteins comprising multiple epitopes (for example from 5 to 100 different epitopes). The polypeptide can optionally include additional segments, for example, it can include at least 4, 5, 10, 15, 20, 25, 30, 40, 50, 60, 75, 90 or even 100 or more segments, each being a part of the naturally occurring E7 protein of a pathogenic agent and/or of a naturally occurring tumor antigen which can be the same or different from the protein or proteins from which the other segments are derived. Each of these segments can have

a length of at least 8 amino acids, and each contains at least one epitope (preferably two or more) different from the epitopes of the other segments. At least one (preferably at least two or three) of the segments in the hybrid polypeptide can contain, for example, 3, 4, 5, 6, 7 or even 10 or more epitopes, particularly epitopes of binding to MHC class I or class II. Two, three or more of the segments can be contiguous in the hybrid polypeptide, i.e., they can be bound end-to-end, without a spacer between them. Alternatively, any two adjacent segments can be bound by a spacer amino acid or a spacer peptide.

Examples of HPV E7-derived antigenic peptides are in Table I.

Table I

| Sequence | SEQ ID NO | Sequence | SEQ ID NO |
|---|---|---|---|
| HEYMLDLQPETTDLY | 2 | KDYILDLQPETTDLH | 27 |
| TLRLCVQSTHVDIRT | 3 | LRTLQQMLLGTLQVV | 28 |
| IRTLEDLLMGTLGIV | 4 | LQQMLLGTLQVVCPG | 29 |
| LEDLLMGTLGIVCPI | 5 | QMLLGTLQVVCPGCA | 30 |
| DLLMGTLGIVCPICS | 6 | VPTLQDVVLELTPQT | 31 |
| KATLQDIVLHLEPQN | 7 | LQDVVLELTPQTEID | 32 |
| IDGVNHQHLPARRAE | 8 | QDVVLELTPQTEIDL | 33 |
| LRAFQQLFLNTLSFV | 9 | CKFVVQLDIQSTKED | 34 |
| FQQLFLNTLSFVCPW | 10 | VVQLDIQSTKEDLRV | 35 |
| QDYVLDLQPEATDLH | 11 | TLHEYMLDL | 36 |
| DIRILQELLMGSFGI | 12 | YMLDLQPET | 37 |
| IRILQELLMGSFGIV | 13 | MLDLQPETT | 38 |
| ELLMGSFGIVCPNCS | 14 | QAEPDRAHYNI | 39 |
| KEYVLDLYPEPTDLY | 15 | QAEPDRAHYNIVTFCCKCD | 40 |
| LRTIQQLLMGTVNIV | 16 | QAEPDRAHYNIVTF | 41 |
| IQQLLMGTVNIVCPT | 17 | QAEPDRAHYNIVT | 42 |
| QLLMGTVNIVCPTCA | 18 | QAEPDRAHYNIVTFCCK | 43 |
| AETLQEIVLHLEPQN | 19 | MLDLQPETTDLYCYEQ | 44 |
| LRTLQQLFLSTLSFV | 20 | MLDLQPETTDLY | 45 |
| LQQLFLSTLSFVCPW | 21 | MLDLQPET | 46 |
| DLRVVQQLLMGALTV | 22 | REYILDLHPEPTDLF | 47 |
| LRVVQQLLMGALTVT | 23 | TCCYTCGTTVRLCIN | 48 |
| VQQLLMGALTVTCPL | 24 | VRTLQQLLMGTCTIV | 49 |
| QQLLMGALTVTCPLC | 25 | LQQLLMGTCTIVCPS | 50 |
| QLLMGALTVTCPLCA | 26 | | |

The person skilled in the art knows of various methods for determining if an HPV E7 peptide is antigenic, for example, the method used in Example 2 (ELISPOT) or the methods described in patent EP1334177-B1 which describe an *in vitro* process for detecting the activity mediated by T lymphocytes of a target antigenic peptide.

In a preferred embodiment, the antigenic peptide or peptides forming part of the conjugate of the invention are derived from the HPV16 E7 protein.

In a particular embodiment of the conjugate of the invention, the latter comprises an HPV E7 protein-derived antigenic peptide corresponding to or containing amino acids 1 to 29 of E7 (SEQ ID NO: 51), an antigenic peptide

corresponding to or containing amino acids 43 to 98 of E7 (SEQ ID NO: 52) or both.

In a particular embodiment of the conjugate of the invention, the EDA region is flanked by two HPV E7 antigenic peptides. In an even more preferred embodiment, the sequence of the fusion protein is SEQ ID NO: 53 or SEQ ID NO:72.

In a particular embodiment component ii) forms a single polypeptide chain or fusion protein with component i).

For the purpose of facilitating the isolation and purification of the fusion protein of the invention, said fusion protein can contain, if desired, an additional peptide which can be used for the purposes of isolating or purifying the fusion protein, such as a tag peptide. Said tag peptide can be located in any position of the fusion protein which does not alter the functionality of any of the polypeptides (i) and (ii). By way of illustration, said tag peptide can be located in the N-terminal position of the conjugate of the invention such that the C-terminal end of the tag peptide is bound to the N-terminal end of the conjugate of the invention. Alternatively, the tag peptide can be located in the C-terminal position of the conjugate of the invention such that the N-terminal end of the tag peptide is bound to the C-terminal end of the conjugate of the invention. Virtually any peptide or peptide sequence allowing the isolation or purification of the fusion protein can be used, for example, polyhistidine sequences, peptide sequences which can be recognized by antibodies which can serve to purify the resulting fusion protein by immunoaffinity chromatography, such as tag peptides, for example, influenza virus hemagglutinin (HA)-derived epitopes (Field et al., 1988, Mol. Cell. Biol., 8: 2159-2165), C-myc and the antibodies 8F9, 3C7, 6E10, G4, B7 and 9E10 against it (Evan et al., 1985, Molecular and Cellular Biology, 5:3610-3616); the Herpes Simplex virus D (gD) tag protein and the antibodies thereof (Paborsky et al., 1990, Protein Engineering, 3:547-553). Other tag peptides include the Flag peptide (Hopp et al., 1988, BioTechnology, 6:1204-1210) and the KT3 epitope (Martin et al., 1993, Science, 255: 192-194). The tag peptide is generally arranged at the amino- or carboxy-terminal end.

A person skilled in the art will appreciate that the different elements of the conjugate of the invention can be placed in any order provided that the fibronectin EDA region maintains its dendritic cell activating properties and that the region or regions of the HPV E7-derived antigenic peptide maintain the antigenic properties.

Thus, examples of arrangement of the elements of the conjugate of the invention, always referring to the placement of elements in the N-terminal to C-terminal direction, are, among others:

- HPV E7-derived antigenic peptide - fibronectin EDA,
- fibronectin EDA - HPV E7-derived antigenic peptide,
- fibronectin EDA - HPV E7-derived antigenic peptide-fibronectin EDA,
- HPV E7-derived antigenic peptide - fibronectin EDA- HPV E7-derived antigenic peptide,
- repeats of the last two.

The person skilled in the art will appreciate that the elements forming the conjugates of the invention, i.e., the EDA peptide and at least one HPV E7-derived antigenic peptide, can be conjugated directly or, alternatively, they can contain an additional amino acid sequence acting as a linker between said components. According to the invention, said intermediate amino acid sequence acts as a hinge region between said domains, allowing them to move independently from one another while maintaining the three-dimensional form of the individual domains. In this sense, a preferred intermediate amino acid sequence according to the invention would be a hinge region characterized by a structural ductility allowing this movement. In a particular embodiment, said intermediate amino acid sequence is a flexible linker. In a preferred embodiment, said flexible linker is a flexible linker peptide with a length of 20 amino acids or less.

The effect of the linker region is to provide space between the EDA peptide and component (ii). It is thus assured that the secondary structure of the EDA peptide is not affected by the presence of component (ii) and vice versa. The spacer is preferably of a polypeptide nature. The linker peptide preferably comprises at least two amino acids, at least three amino acids, at least five amino acids, at least ten amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids or approximately 100 amino acids.

In a more preferred embodiment, the linker peptide comprises 2 or more amino acids selected from the group consisting of glycine, serine, alanine and threonine. In a preferred embodiment of the invention, said flexible linker is a polyglycine linker. The possible examples of linker/spacer sequences include SGGTSGSTSGTGST (SEQ ID NO: 54), AGSSTGSSTGPGSTT (SEQ ID NO: 55) or GGSGGAP (SEQ ID NO: 56). These sequences have been used for binding designed coiled coils to other protein domains (Muller, K.M., Arndt, K.M. and Alber, T., Meth. Enzimology, 2000, 328: 261-281). Preferably, said linker comprises or consists of amino acid sequence GGGVEGGG (SEQ ID NO: 57).

The linker can be bound to components flanking the two components of the conjugates of the invention by means of covalent bonds and preferably the spacer is essentially non-immunogenic, and/or is not prone to proteolytic cleavage, and/or does not comprise any cysteine residue. Similarly, the three-dimensional structure of the spacer is preferably linear or substantially linear.

The preferred examples of spacer or linker peptides include those that have been used to bind proteins without substantially deteriorating the function of the bound proteins or at least without substantially deteriorating the function

of one of the bound proteins. More preferably the spacers or linkers have been used to bind proteins comprising coiled coil structures.

The linker can include tetranectin residues 53-56, which in tetranectin forms a β-sheet, and residues 57-59 forming a turn in tetranectin (Nielsen, B.B. et al., FEBS Lett. 412: 388-396, 1997). The sequence of the segment is GTKVHMK (SEQ ID NO: 58). This linker has the advantage that when it is present in the native tetranectin, it is binding the trimerization domain with the CRD domain, and therefore it is suitable for connecting the trimerization domain to another domain in general. Furthermore the resulting construct is not expected to be more immunogenic than the construct without a linker.

Alternatively, a suitable linker peptide can be based on the sequence of 10 amino acid residues of the upper hinge region of murine IgG3. This peptide (PKPSTPPGSS, SEQ ID NO: 59) has been used for the production of dimerized antibodies by means of a coiled coil (Pack P. and Pluckthun, A., 1992, Biochemistry 31:1579-1584) and can be useful as a spacer peptide according to the present invention. Even more preferably, it can be a corresponding sequence of the upper hinge region of human IgG3. The sequences of human IgG3 are not expected to be immunogenic in human beings. Additional linker peptides that can be used in the conjugate of the invention include the peptide of sequence APAETKAEPMT (SEQ ID NO: 60) and the peptide of sequence GAP.

Alternatively, the two components of the conjugates of the invention can be connected by a peptide the sequence of which contains a cleavage target for a protease, thus allowing the separation of the EDA peptide of component (ii). The protease cleavage sites suitable for their incorporation in the polypeptides of the invention include the enterokinase target site (sequence DDDDK SEQ ID NO: 61), factor Xa target site (cleavage site IEDGR, SEQ ID NO: 62), thrombin target site (cleavage site LVPRGS, SEQ ID NO: 63), protease TEV target site (cleavage site ENLYFQG, SEQ ID NO: 64), PreScission protease target site (cleavage site LEVLFQGP, SEQ ID NO: 65) and intein target site and the like.

<u>Methods for obtaining the conjugates of the invention</u>

The conjugates of the invention can be obtained using any method known for a person skilled in the art. It is thus possible to obtain the EDA peptide or the variant of said protein by any standard method. For example, the EDA peptide can be obtained from cDNA by means of expression in a heterologous organism such as, for example, *Escherichia coli, Sacharomyces cerevisiae, Pichia pastoris.*

Once a sufficient amount of the purified EDA peptide is available, the latter must be conjugated to the HPV E7-derived antigenic peptide or peptides. The conjugation of component (ii) to the EDA molecule can be carried out in different ways. One possibility is the direct conjugation of a functional group to the therapeutically active component in a position which does not interfere with the activity of said component. As understood in the present invention functional groups refer to a group of specific atoms in a molecule which are responsible for a characteristic chemical reaction of said molecule. Examples of functional groups include, without limitation, hydroxy, aldehyde, alkyl, alkenyl, alkynyl, amide, carboxamide, primary, secondary, tertiary and quaternary amines, aminoxy, azide, azo (diimide), benzyl, carbonate, ester, ether, glyoxylyl, haloalkyl, haloformyl, imine, imide, ketone, maleimide, isocyanide, isocyanate, carbonyl, nitrate, nitrite, nitro, nitroso, peroxide, phenyl, phosphine, phosphate, phosphono, pyridyl, sulfide, sulfonyl, sulfinyl, thioester, thiol and oxidized 3,4-dihydroxyphenylalanine (DOPA) groups. Examples of said groups are groups maleimide or glyoxylyl which react specifically with thiol groups in the Apo A molecule and oxidized 3,4-dihydroxyphenylalanine (DOPA) groups which react with primary amino groups in the EDA molecule and of component (ii).

Another possibility is to conjugate component (ii) to the EDA molecule by means of the use of homo- or heterobifunctional groups. The bifunctional group can first be conjugated to the therapeutically active compound and, then, conjugated to the EDA peptide or, alternatively, it is possible to conjugate the bifunctional group to the EDA peptide and, then, conjugate the latter to component (ii). Illustrative examples of this type of conjugates include the conjugates known as ketone-oxime (described in US20050255042) in which the first component of the conjugate comprises an aminoxy group which is bound to a ketone group present in a heterobifunctional group which, in turn, is bound to an amino group in the second component of the conjugate.

In another embodiment, the agent used to conjugate components (i) and (ii) of the conjugates of the invention can be photolytically, chemically, thermically or enzymatically processed. In particular, the use of linking agents which can be hydrolyzed by enzymes which are in the target cell, such that the therapeutically active compound is only released into the cell is of interest. Examples of linking agent types which can be intracellularly processed have been described in WO04054622, WO06107617, WO07046893 and WO07112193.

In a preferred embodiment, since component (ii) of the conjugate of the invention is a compound of a peptide nature, including both oligopeptides and peptides, methods for chemically modifying a polypeptide chain which are widely known for the person skilled in the art and include methods based on the conjugation through the thiol groups present in the cysteine moieties, methods based on the conjugation through the primary amino groups present in the lysine moieties (US6809186), methods based on the conjugation through the N- and C-terminal moieties can be used. Reagents suitable for the modification of polypeptides to allow their coupling to other compounds include: glutaraldehyde

(allows binding compounds to the N-terminal end of polypeptides), carbodiimide (allows binding the compound to the C-terminal end of a polypeptide), succinimide esters (for example MBS, SMCC) which allow activating the N-terminal end and cysteine moieties, benzidine (BDB), which allows activating tyrosine moieties, periodate, which allows activating carbohydrate moieties in those proteins which are glycosylated.

Polynucleotides, gene constructs, vectors and host cells of the invention

In the particular event that the EDA component and the HPV E7-derived antigenic peptide or peptides form a single peptide chain, the expression of the conjugate in a single step using a polynucleotide encoding said conjugate is possible. Thus, in another aspect, the invention relates to a polynucleotide encoding the conjugate of the invention. The person skilled in the art will appreciate that the polynucleotides of the invention will encode only those conjugates in which component (ii) and the EDA polypeptide or its functionally equivalent variant form a single peptide chain, independently of the relative orientation and independently of the fact that both components are directly linked or separated by a spacer region.

As used in the present invention, the term "polynucleotide" refers to a polymeric form of nucleotides of any length and formed by ribonucleotides and/or deoxyribonucleotides. The term includes both single-stranded and double-stranded polynucleotides, as well as modified polynucleotides (methylated, protected and the like).

In another aspect, the invention relates to a gene construct, hereinafter gene construct of the invention, which comprises a polynucleotide of the invention. The construct preferably comprises the polynucleotide of the invention operatively bound to sequences regulating the expression of the polynucleotide of the invention. In principle, any promoter can be used for the gene constructs of the present invention provided that said promoter is compatible with the cells in which the polynucleotide is to be expressed. Thus, promoters suitable for performing the present invention include, without necessarily being limited to, constitutive promoters such as those derived from the genomes of eukaryotic viruses such as the polyoma virus, adenovirus, SV40, CMV, avian sarcoma virus, hepatitis B virus, the metallothionein gene promoter, the herpes simplex virus thymidine kinase gene promoter, retrovirus LTR regions, the immunoglobulin gene promoter, the actin gene promoter, the EF-1alpha gene promoter as well as inducible promoters in which the expression of the protein depends on adding a molecule or an exogenous signal, such as the tetracycline system, the NFκB/UV light system, the Cre/Lox system and the heat shock gene promoter, the regulatable RNA polymerase II promoters described in WO/2006/135436 as well as tissue-specific promoters.

Other examples of promoters which are tissue-specific include the albumin gene promoter (Miyatake et al., 1997, J. Virol, 71:5124-32), the hepatitis virus core promoter (Sandig et al., 1996, Gene Ther., 3:1002-9); the alpha-fetoprotein gene promoter (Arbuthnot et al., 1996, Hum.GeneTher., 7:1503-14), and thyroxine-binding globulin-binding protein gene promoter (Wang, L., et al., 1997, Proc.Natl.Acad.Sci.USA 94:11563-11566). The constructs of the invention preferably contain dendritic cell-specific promoters such as the CD11c promoter (Masood, R., et al. 2001. Int J Mol Med 8:335-343; Somia, N.V., et al. 1995. Proc Acad Sci USA 92:7570-7574), the fascin promoter (Sudowe, S., et al., 2006. J Allergy Clin Immunol. 117:196-203), the CD83 gene promoter, the CD36 gene promoter or the Dectin-2 promoter (Gene Ther., 2001, 8:1729-1737).

The polynucleotides of the invention or the gene constructs forming them can form part of a vector. Thus, in another aspect, the invention relates to a vector which comprises a polynucleotide or a gene construct of the invention. The person skilled in the art will appreciate that there is no limitation regarding the type of vector which can be used since said vector can be a cloning vector suitable for propagation and for obtaining the suitable polynucleotides or gene constructs or expression vectors in different heterologous organisms suitable for the purification of the conjugates. Thus, suitable vectors according to the present invention include expression vectors in prokaryotes such as pUC18, pUC19, Bluescript and the derivatives thereof, mp18, mp19, pBR322, pMB9, ColEl, pCRl, RP4, phages and shuttle vectors such as pSA3 and pAT28, expression vectors in yeasts such as 2-micron plasmid type vectors, integration plasmids, YEP vectors, centromeric plasmids and the like, expression vectors in insect cells such as the vectors of the pAC series and of the pVL series, expression vectors in plants such as vectors of the pIBI, pEarleyGate, pAVA, pCAMBIA, pGSA, pGWB, pMDC, pMY, pORE series and the like and expression vectors in superior eukaryotic cells either based on viral vectors (adenoviruses, viruses associated to adenoviruses as well as retroviruses and lentiviruses) as well as non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAXl, pZeoSV2, pCI, pSVL and pKSV-10, pBPV-1, pML2d and pTDTl.

The vector of the invention can be used to transform, transfect or infect cells which can be transformed, transfected or infected by said vector. Said cells can be prokaryotic or eukaryotic cells. By way of example, the vector in which said DNA sequence is introduced can be a plasmid or a vector which, when it is introduced in a host cell, is integrated in the genome of said cell and replicates together with the chromosome (or chromosomes) in which it has been integrated. Said vector can be obtained by conventional methods known by the persons skilled in the art (Sambrook et al., 2001, "Molecular cloning, to Laboratory Manual", 2nd ed., Cold Spring Harbor Laboratory Press, N.Y. Vol 1-3 a).

Therefore, in another aspect, the invention relates to a cell comprising a polynucleotide, a gene construct or a

vector of the invention, for which said cell could have been transformed, transfected or infected with a construct or vector provided by this invention. Transformed, transfected or infected cells can be obtained by conventional methods known by the persons skilled in the art (Sambrook et *al.,* 2001, mentioned above). In a particular embodiment, said host cell is an animal cell transfected or infected with a suitable vector.

Host cells suitable for the expression of the conjugates of the invention include, mammalian, plant, insect, fungal and bacterial cells. Bacterial cells include, Gram-positive bacterial cells such as species of the *Bacillus, Streptomyces and Staphylococcus* genera and Gram-negative bacterial cells such as cells of the *Escherichia* and *Pseudomonas* genera. Fungal cells preferably include yeast cells such as *Saccharomyces, Pichia pastoris* and *Hansenula polymorpha.* Insect cells include, without limitation, Drosophila cells and Sf9 cells. Plant cells include, among others, crop plant cells such as cereal, medicinal, ornamental or bulbous plants. Mammalian cells suitable for the present invention include epithelial cell lines (porcine, etc.), osteosarcoma cell lines (human, etc.), cell lines of neuroblastoma (human, etc.), epithelial carcinomas (human, etc.), glial cells (murine, etc.), liver cell lines (from monkeys, etc.), CHO (Chinese Hamster Ovary) cells, COS cells, BHK cells, HeLa cells, 911 cells, AT1080 cells , A549 cells, 293 cells or PER.C6 cells, human ECC NTERA-2 cells, D3 cells of the mESC line, human embryonic stem cells such as HS293 and BGV01, SHEF1, SHEF2 and HS181, NIH3T3 cells, 293T cells, REH cells and MCF-7 cells and hMSC cells.

Immunogenic compositions of the invention.

The inventors have observed that the combination of the conjugate of the invention and of a Toll-like receptor (TLR) agonist allowed generating an immune response greater than that obtained when each of said components was separately administered. Thus, as observed in Example 3, the compositions formed by the EDA-HPVE7 fusion protein and polyI:C are capable of eradicating tumors which are not treatable when only the fusion protein is administered.

Thus, in another aspect the invention relates to a composition (hereinafter composition one of the invention or first composition of the invention) comprising, together or separately:

(i) a conjugate of the invention, a polynucleotide or gene construct of the invention, a vector of the invention, a host cell according to the invention and
(ii) a TLR ligand.

The molar concentrations of the components forming part of the first composition of the invention can vary, but preferably include ratios of the two components between 50:1 and 1:50, more preferably between 20:1 and 1:20, between 1:10 and 10:1, between 5:1 and 1:5.

The first component i) of the composition has been described in detail in the context of the peptide of the invention. In a preferred particular embodiment, said first component comprises the fibronectin EDA region of human origin. In another embodiment, the first component of the composition of the invention comprises amino acids 1 to 29 of the E7 protein and/or amino acids 43 to 98 of the E7 protein. In an even more preferred embodiment, the first component of the conjugate forming part of the composition of the invention comprises the EDA region flanked by two HPV E7 antigenic peptides, as described in SEQ ID NO: 53 (that additionally comprises a 6 histidine tag) or SEQ ID NO:72.

In the present invention "TLR receptor ligand" is understood as a molecule which specifically binds to at least one of the TLR (toll-like receptor) receptors and which upon binding is capable of stimulating some of the signals of co-stimulation signals characteristic of the binding of said receptor with its natural ligand or other signals which result from the binding of said receptor with a TLR agonist.

Toll-like receptors (or TLRs) are a family of type I transmembrane proteins forming part of the innate immune system. In vertebrates they also enable the adaptation of the immune system. TLRs together with interleukin receptors form a superfamily known as the Interleukin-1/toll-like receptor superfamily. All the members of this family have in common the domain called the Toll-IL-1 receptor (TIL) domain.

It has been estimated that most mammals have between 10 and 15 types of TLRs. Thirteen types of TLRs have been identified up until now in human and mice (Du X, et al. 2000, Eur.Cytokine Netw. 11: 362-71; Chuang TH. et al., 2000. Eur. Cytokine Netw. 11: 372-378; Tabeta K, et al.; 2004, Proc. Natl. Acad. Sci. U.S.A. 101:3516-3521).

TLR ligands induce several immune responses depending on the cells in which the TLR is expressed as well as depending on the origin of TLR ligand. For example, in the case of microbial ligands, immune cells can produce cytokines which will cause inflammation. In the case of a viral factor, the cells can undergo apoptosis.

In a particular embodiment, the ligands are agonist ligands. Agonist ligands of TLR receptors are (i) natural ligands of the actual TLR receptor, or a functionally equivalent variant thereof which conserves the capacity to bind to the TLR receptor and induce co-stimulation signals thereon, or (ii) an agonist antibody against the TLR receptor, or a functionally equivalent variant thereof capable of specifically binding to the TLR receptor and, more particularly, to the extracellular domain of said receptor, and inducing some of the immune signals controlled by this receptor and associated proteins. The binding specificity can be for the human TLR receptor or for a TLR receptor homologous to the human

one of a different species.

Examples of ligands of the different TLRs are schematized in Table 2.

| TLR (No.) | Ligand |
|---|---|
| TLR 1 | Multiple triacyl Lipopeptides. |
| TLR 2 | Multiple glycopeptides, lipopeptides and lipoproteins. Lipoteichoic acid, HSP70, zymosan from host cells. |
| TLR 3 | double-stranded RNA, poly I:C (polyinosinic-polycytidylic acid or polyinosinic-polycytidylic acid sodium salt) |
| TLR 4 | Lipopolysaccharides (Gram-negative bacteria), different heat shock proteins (bacteria and host cells), fibrinogen from host cells, heparan sulfate fragments from host cells, hyaluronic acid fragments from host cells, many others. |
| TLR 5 | Flagellin |
| TLR 6 | multiple diacyl lipopeptides (mycoplasmas) |
| TLR 7 | Imidazoquinoline, loxoribine (guanosine analog), small synthetic compounds of bropirimine and single-stranded RNA. |
| TLR 8 | Small synthetic compounds, single-stranded RNA. |
| TLR 9 | Unmethylated CpG DNA (bacteria) |
| TLR 11 | Profilin *(Toxoplasma gondii*) |

As the person skilled in the art understands, there is a large variety of immune assays available to detect the activity of agonist ligands and generally ligands of the TLR receptor, such as the *in vitro* co-stimulation of dendritic cells. Briefly, said assay consists of contacting a culture of dendritic cells with a TLR agonist ligand and measuring the activation of said cells. Said activation can be determined by means of the detection of any marker, for example poly(I:C) in the event that the receptor is TLR3. The activated dendritic cells express different proteins such as CD80 (B7.1), CD86 (B7.2) and CD40. It is thus possible to detect the agonistic activity of a TLR agonist ligand by means of detecting changes in the expression levels of said proteins in the dendritic cells after being exposed to said ligand, as described for example by Chen X.Z. et al. (Arch Dermatol Res. 2009 Jul 4 Epub ahead of print).

In another preferred particular embodiment, the TLR ligand is selected from the group of a TLR3 ligand, a TLR9 ligand and a combination of both. In a more preferred manner, the TLR3 ligand is poly(I:C) (polyinosinic-polycytidylic acid or polyinosinic-polycytidylic acid sodium salt).

In another preferred embodiment, the TLR9 ligand is an oligonucleotide comprising at least one CpG motif more preferably, type B CpG-phosphorothioate (CpG 1826: 5'-TCCATGACGTTCCTGACGTT-3' SEQ ID NO: 66).

The authors of the present invention have observed that the conjugates of the invention are capable of improving the antitumor response obtained by means of the use of a TLR ligand and of a chemotherapeutic agent. Specifically, Figure 7 of the invention shows how the administration of a composition comprising the EDA-HPVE7 fusion protein, the TLR9 ligand CpG and cyclophosphamide results in a greater tumor size reduction than the administration of cyclophosphamide and the TLR9 ligand.

Thus, in another aspect the invention relates to a composition of the invention (hereinafter, composition two of the invention or second composition of the invention) comprising:

(i) a conjugate of the invention, a polynucleotide or gene construct of the invention, a vector of the invention, a host cell according to the invention,
(ii) a TLR ligand and
(iii) a chemotherapeutic agent.

Components (i) (conjugate of the invention) and (ii) (the TLR ligand) of the second composition of the invention have been described in detail in previous sections.

"Chemotherapeutic agent" is understood as any substance which is capable of inhibiting cell proliferation without necessarily killing the cell or which is capable of inducing cell death. The agents capable of inhibiting cell proliferation without causing cell death are generically called cytostatic agents, whereas those which are capable of inducing cell death normally by means of activating apoptosis are generically called cytotoxic agents. Examples of chemotherapeutic

agents suitable for their use in the compositions of the invention include (i) microtubule-stabilizing agents such as taxanes, paclitaxelm, docetaxel, epothilones and laulimalides, (ii) kinase inhibitors such as Iressa(R), Gleevec, Tarceva™, (Erlotinib HCl), BAY-43-9006, (iii) antibodies specific for receptors with kinase activity including, Trastuzumab (Herceptin(R)), Cetuximab (Erbitux(R)), Bevacizumab (Avastin™), Rituximab (ritusan(R)), Pertuzumab (Omnitarg™); (iv) mTOR pathway inhibitors such as rapamycin and CCI-778; (v) Apo2L/Trail, (vi) anti-angiogenic agents such as endostatin, combretastatin, angiostatin, thrombospondin and vascular endothelial growth inhibitor (VEGI); (vii) antineoplastic vaccines including activated T cells, unspecific immunostimulating agents (for example interferons, interleukins); (viii) antibiotic cytotoxic agents such doxorubicin, bleomycin, dactinomycin, daunorubicin, epirubicin, mitomycin and mitozantrone; (ix) alkylating agents such as Melphalan, Carmustine, Lomustine, cyclophosphamide, ifosfamide, Chlorambucil, Fotemustine, Busulfan, Temozolomide and thiotepa; (x) antineoplastic hormonal agents such as Nilutamide, cyproterone acetate, anastrozole, Exemestane, Tamoxifen, Raloxifene, Bicalutamide, Aminoglutethimide, leuprorelin acetate, Toremifene citrate, Letrozole, Flutamide, Megestrol acetate and goserelin acetate; (xi) gonadal hormones such as cyproterone acetate and medroxyprogesterone acetate; (xii) antimetabolites such as Cytarabine, Fluorouracil, Gemcitabine, Topotecan, Hydroxyurea, Thioguanine, Methotrexate, Colaspase, Raltitrexed and capecitabine; (xiii) anabolic agents such as nandrolone; (xiv) adrenal steroid hormones such as methylprednisolone acetate, dexamethasone, hydrocortisone, prednisolone and prednisone; (xv) antineoplastic agents such as Carboplatin, Cisplatin, Oxaliplatin, Etoposide and Dacarbazine and (xvi) topoisomerase inhibitors such as topotecan and irinotecan.

Without wishing to be bound by any theory, it is believed that chemotherapeutic agents, when administered in suitable doses, can act as immunostimulating agents indirectly by means of the inactivation of regulatory T cells.

In a preferred embodiment, the chemotherapeutic agent is a cytostatic agent, more preferably it is cyclophosphamide or a cyclophosphamide analog. Cyclophosphamide analogs are well known in the literature (Cyclophosphamide, Merck Index, 11th Edition, pages 429-430; document US5190929).

Hereinafter, the term "composition of the invention" will be occasionally mentioned to refer to both to the first composition of the invention and to the second composition of the invention. As a person skilled in the art understands, the compositions of the invention can be formulated as a single component or alternatively presented as separate formulations which can be combined for their subsequent administration. The compositions of the invention can also be presented as parts of a kit, in which each of the components is formulated separately but packaged in a single container.

<u>Medical uses of the conjugates and compositions of the invention</u>

The investigators have observed that the conjugates of the invention are capable of inducing the maturation of dendritic cells, inducing the activation of the antitumor immune response *in vivo* against the peptide and of eradicating large and well-established tumors expressing the HPVE7 protein (see Examples 1 to 3 of the invention).

Therefore, in another aspect, the invention relates to a conjugate of the invention, a polynucleotide of the invention, a gene construct of the invention, a vector of the invention or a composition of the invention for its use in medicine.

In another aspect, the invention relates to a pharmaceutical composition, or pharmaceutical composition of the invention, comprising a fusion protein of the invention, a polynucleotide of the invention, a vector of the invention, a cell of the invention, a composition of the invention and at least one pharmacologically acceptable carrier or adjuvant.

"Adjuvant" is understood as any substance intensifying the effectiveness of the pharmaceutical composition of the invention.

The examples of adjuvants include, without limitation, adjuvants formed by aluminum salts (alum), such as aluminum hydroxide, aluminum phosphate, aluminum sulfate, etc, formulations of oil-in-water or water-in-oil emulsions such as complete Freund's Adjuvant (CFA) as well as the incomplete Freund's Adjuvant (IFA); mineral gels; block copolymers, Avridine™, SEAM62, adjuvants formed by components of the bacterial cell wall such as adjuvants including liposaccharides (e.g., lipid A or Monophosphoryl Lipid A (MLA), trehalose dimycolate (TDM), and components of the cell wall skeleton (CWS), heat shock proteins or the derivatives thereof, adjuvants derived from ADP-ribosylating bacterial toxins, which include diphtheria toxin (DT), pertussis toxin (PT), cholera toxin (CT), *E.coli* heat-labile toxins (LT1 and LT2), *Pseudomonas* Endotoxin A and exotoxin, *B. cereus* exoenzyme B, *B. sphaericus* toxin, C. *botulinum* toxins C2 and C3, *C. limosum* exoenzyme as well as the toxins of *C. perfringens, C. spiriforma* and C. *difficile, S. aureus,* EDIM and mutants of mutant toxins such as CRM-197, nontoxic mutants of diphtheria toxin; saponins such as ISCOMs (immunostimulating complexes), chemokines, quimiokines and cytokines such as interleukins (IL-I IL-2, IL-4, IL-5, IL-6, IL-7, IL-8, IL- 12, etc), interferons (such as the interferon gamma) macrophage colony stimulating factor (M-CSF), Tumor necrosis factor (TNF), defensins 1 or 2, RANTES, MIPl-alpha, and MEP-2, muramyl peptides such as N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-s-n-glycero-3-hydroxyphosphoryloxy)-ethylamine (MTP-PE) etc; adjuvants derived from the family of CpG molecules, CpG dinucleotides and synthetic oligonucleotides which comprise CpG motifs, *C. limosum* exoenzyme and synthetic adjuvants such as PCPP, the cholera toxin, *Salmonella* toxin, alum and the like, aluminum hydroxide, N-acetyl-muramyl-L-threonyl-D-isoglutamine (thr-MDP), N-acetyl-nor-muramyl-L-alanyl-D-isoglutamine, MTP-PE and RIBI, containing three components extracted from bacteria, monophosphoryl lipid A, trehalose dimycolate and cell wall skeleton (MPL+TDM+CWS) in a squalene emulsion at 2%/Tween 80. Other examples of adjuvants include DDA (dimethyl dioctadecyl ammonium bromide), complete and incomplete Freund's adjuvants and QuilA.

The term "carrier" refers to a diluent or excipient with which the active ingredient is administered. Such pharmaceutical carriers can be sterile liquids, such as water and oils, including those of petroleum, animal, plant or synthetic origin, such as peanut oil, soybean oil, mineral oil, sesame oil and the like. Water or aqueous solutions of saline solution and aqueous dextrose and glycerol solutions, particularly for injectable solutions, are preferably used as carriers. Suitable pharmaceutical carriers are described in "Remington's Pharmaceutical Sciences" by E.W. Martin, 1995. Preferably, the carriers of the invention are approved by a regulatory agency of the Federal or a state government or listed in the United States Pharmacopoeia or another generally recognized pharmacopeia for use in animals, and more particularly in humans.

The carriers and the auxiliary substances necessary to manufacture the desired pharmaceutical dosage form of the pharmaceutical composition of the invention will depend, among others factors, on the pharmaceutical dosage form chosen. Said pharmaceutical dosage forms of the pharmaceutical composition will be manufactured according to conventional methods known by the person skilled in the art. A review of different administration methods for active ingredients, excipients which are to be used and processes for producing them can be found in "Tratado de Farmacia Galénica", C. Faulí i Trillo, Luzán 5, S.A. de Ediciones, 1993. Examples of pharmaceutical compositions include any solid (tablets, pills, capsules, granulates, etc.) or liquid (solutions, suspensions or emulsions) composition for oral, topical or parenteral administration. Furthermore, the pharmaceutical composition can contain, as appropriate, stabilizers, suspensions, preservatives, surfactants and the like.

For use in medicine, the conjugates of the invention can be found in the form of prodrug, salt, solvate or clathrate, either isolated or in combination with additional active agents. The combinations of compounds according to the present invention can be formulated together with an excipient which is acceptable from the pharmaceutical point of view. Preferred excipients for their use in the present invention include sugars, starches, celluloses, gums and proteins. In a particular embodiment, the pharmaceutical composition of the invention will be formulated in a solid (for example tablets, capsules, coated tablets, granulates, suppositories, sterile crystalline or amorphous solids which can be reconstituted to provide liquid forms, etc.), liquid (for example solutions, suspensions, emulsions, elixirs, lotions, unguents etc.) or semisolid (gels, pomades, creams and the like) pharmaceutical dosage form. The pharmaceutical compositions of the invention can be administered by any route, including, oral, intravenous, intramuscular, intrarterial, intramedullary, intrathecal, intraventricular, transdermal, subcutaneous, intraperitoneal, intranasal, enteral, topical, sublingual or rectal route. A review of the different forms for the administration of active ingredients, of the excipients to be used and of the processes for manufacturing them can be found in Tratado de Farmacia Galénica, C. Faulí i Trillo, Luzán 5, S.A. de Ediciones, 1993 and in Remington's Pharmaceutical Sciences (A.R. Gennaro, Ed.), 20th edition, Williams & Wilkins PA, USA (2000). Examples of pharmaceutically acceptable carriers are known in the state of the art and include phosphate buffered saline solutions, water, emulsions, such as oil/water emulsions, different types of wetting agents, sterile solutions, etc. The compositions comprising said carriers can be formulated by conventional methods known in the state of the art.

In the event that nucleic acids (the polynucleotides of the invention, the vectors or the gene constructs) are administered, the invention contemplates pharmaceutical compositions especially prepared for the administration of said nucleic acids. The pharmaceutical compositions can comprise said nucleic acids in naked form, i.e., in the absence of compounds protecting the nucleic acids from their degradation by the nucleases of the organism, involving the advantage of eliminating the toxicity associated to the reagents used for the transfection. Administration routes suitable for the naked compounds include intravascular, intratumoral, intracranial, intraperitoneal, intrasplenic, intramuscular, subretinal, subcutaneous, mucosal, topical and oral route (Templeton, 2002, DNA Cell Biol., 21:857-867). Alternatively, the nucleic acids can be administered forming part of liposomes, conjugated to cholesterol or conjugated to compounds capable of promoting the translocation through cell membranes such as the Tat peptide derived from HIV-1 TAT protein, the third helix of the homeodomain of the *D. melanogaster* Antennapedia protein, the herpes simplex virus VP22protein, arginine oligomers and peptides such as those described in WO07069090 (Lindgren, A. et al., 2000, Trends Pharmacol. Sci, 21:99-103, Schwarze, S.R. et al., 2000, Trends Pharmacol. Sci., 21:45-48, Lundberg, M et al., 2003, Mol Therapy 8:143-150 and Snyder, E.L. and Dowdy, S.F., 2004, Pharm. Res. 21:389-393). Alternatively, the polynucleotide can be administered forming part of a plasmid vector or of a viral vector, preferably vectors based on adenoviruses, on adeno-associated viruses or on retroviruses, such as viruses based on the murine leukemia virus (MLV) or on lentiviruses (HIV, FIV, EIAV).

The compositions of the invention can be administered in doses of less than 10 mg per kilogram of body weight, preferably less than 5, 2, 1, 0.5, 0.1, 0.05, 0.01, 0.005, 0.001, 0.0005, 0.0001, 0.00005 or 0.00001 mg per each kg of body weight. The unit dose can be administered by injection, by inhalation or by topical administration.

The dose depends on the severity and response of the condition to be treated and can vary between several days and several months or until observing that the condition remits. The optimum dosage can be determined performing

periodic measurements of the concentrations of agent in the organism of the patient. The optimum dose can be determined from the EC50 values obtained by means of previous *in vitro* or *in vivo* assays in animal models. The unit dose can be administered once a day or less than once a day, preferably, less than once a day every 2, 4, 8 or 30 days. Alternatively, it is possible to administer an initial dose followed by one or several maintenance doses, generally of a lower amount than the initial dose. The maintenance regimen can involve treating the patient with doses ranging between 0.01 μg and 1.4 mg/kg of body weight per day, for example 10, 1, 0.1, 0.01, 0.001, or 0.00001 mg per kg of body weight per day. The maintenance doses are preferably administered at most once a day every 5, 10 or 30 days. The treatment must be continued during a time which will vary according to the type of disorder that the patient suffers from, its severity and the condition of the patient. After the treatment, the evolution of the patient must be monitored to determine if the dose should be increased in the event that the disease does not respond to the treatment or the dose should be reduced if an improvement of the disease is observed or if undesirable side effects are observed.

In a particular embodiment, the components of the compositions of the invention can be administrated in a simultaneously, sequentially or separately. In a preferred embodiment, the chemotherapy agent (preferably cyclophosphamide) is first administrated and subsequently, after a variable time period, the rest of the components of the compositions of the invention are administrated, i.e. conjugate of the invention, polynucleotide or gene construct of the invention, vector of the invention or host cell according to the invention and the TLR ligand. The administration of the conjugate of the invention, the polynucleotide or gene construct of the invention, the vector of the invention or the host cell according to the invention and the TLR can be done simultaneously, separately or sequentially. In a preferred embodiment, the TLR ligand (preferably pI:C or CpG-B/DOTAP) and the conjugated, polynucleotide, gene construct, vector or host cell according to the invention (preferably the fusion protein) are administrated simultaneously. In the event that the compositions of the invention comprise a chemotherapeutic agent and, specifically, cyclophosphamide (CPA), in a preferred embodiment, CPA is used at a suboptimal concentration, called "metronomic dose", which is capable of selectively killing the Treg cells (Ghiringhelli et al., Cancer Immunol. Immunother.2007, 56:641-8).

The compositions of the invention can be administrated as a unitary dose or, alternatively, as a first dose and one or more booster doses. Thus, in a preferred embodiment, the compositions of the invention are administrated as a first dose and a second booster dose. When the compositions of the invention are administrated separately during time, it is understood that a first dose of the first component of the invention, a second dose of the second component of the invention, a second booster dose from the first component of the invention, a second booster dose from the second component of the invention and so successively are administrated during time. In a more preferred embodiment, the therapy includes a first administration wherein the chemotherapy agent (preferably cyclophosphamide) is administrated, a second administration wherein the conjugate, polynucleotide, genetic construct, vector or host cell according to the invention and the TLR ligand are administrated; a third administration that includes the chemotherapy agent (preferably cyclophosphamide) and a fourth administration wherein the conjugate, polynucleotide, genetic construct, vector or host cell according to the invention, together with the TLR ligand (preferably pI:C or CpG-B/DOTAP), is again administrated. In the second and fourth administrations, the TLR ligand (preferably pI:C or CpG-B/DOTAP) and the conjugate, polynucleotide, genetic construct, vector or host cell according to the invention (preferably the fusion protein) is administrated simultaneously, separately or sequentially.

In a preferred embodiment, the TRL ligand (preferably pI:C or CpG-B/DOTAP) and the conjugate, polynucleotide, genetic construct, vector or host cell according to the invention (preferably the fusion protein) are administrated simultaneously.

The daily dose can be administered in a single dose or in two or more doses according to the particular circumstances. If a repeated administration or frequent administrations are desired the implantation of an administration device such as a pump, a semi-permanent catheter (intravenous, intraperitoneal, intracisternal or intracapsular) or a reservoir is recommended.

In another aspect, the invention relates to the use of a conjugate of the invention, a polynucleotide of the invention, a gene construct of the invention, a vector of the invention, a cell of the invention, a composition of the invention or a pharmaceutical composition of the invention for the manufacture of a vaccine. In other words, the invention relates to a conjugate of the invention, a polynucleotide of the invention, a gene construct of the invention, a vector of the invention, a cell of the invention, a composition of the invention or pharmaceutical composition of the invention for its use as vaccine.

"Vaccine" is understood as an antigen preparation which once inside the organism causes a response to attack, called antibody. This response generates immunological memory causing, in most cases, permanent immunity.

The vaccine is systematically or locally administered. The vaccine can be administered by means of a single administration, or with a boost by means of multiple administrations as has been previously described for the administration of the compositions of the invention.

In another aspect, the invention relates to the use of a conjugate of the invention, a polynucleotide of the invention, a gene construct of the invention, a vector of the invention, a cell of the invention, a composition of the invention or a pharmaceutical composition of the invention for the manufacture of a medicament for the prevention and the treatment

of an infection caused by the human papilloma virus and/or of cervical cancer associated with HPV infections. Alternatively, the invention relates to a conjugate of the invention, a polynucleotide of the invention, a gene construct of the invention, a vector of the invention, a cell of the invention, a composition of the invention or a pharmaceutical composition of the invention for their use in the prevention and the treatment of an infection caused by the human papilloma virus and/or cervical cancer associated with HPV infections.

Infections and diseases caused by the human papilloma virus include warts (such as foot warts), genital warts, recurrent respiratory papillomatosis (such as laryngeal papillomas) and cancers associated with papilloma infections. Cancers which have been associated with the papilloma virus include anogenital cancers (e.g. cervical, perianal, vulvar, vaginal, penile cancers, etc), head and neck cancers (e.g. oropharyngeal cavity cancer, esophageal cancer, etc) and skin cancers (e.g., basal cell carcinoma, squamous cell carcinoma).

Methods of vaccination with dendritic cells of the invention

Another cancer therapy approach is to take advantage of the normal dendritic cell role as an immune educator. As has been previously mentioned, dendritic cells capture virus antigens among others and present them to T cells to recruit their help in an initial T cell immune response. This works well against foreign cells entering the body, but cancer cells frequently evade the "self"/"foreign" substance detection system. The investigators, by modifying the dendritic cells, are capable of activating a special type of autoimmune response which includes an attack of T cells against cancer cells. Due to the fact that a tumor agent alone is not enough to generate an immune response, it is possible to contact an immature dendritic cell with a conjugate of the invention, a polynucleotide of the invention, a cell of the invention, a composition of the invention or a pharmaceutical composition of the invention, which results in the activation of the dendritic cells, the capture of the HPV E7-derived antigen or antigens and the presentation thereof in the surface associated to the major histocompatibility antigen. These cells thus activated can be administered to the patient, such that the presentation of the tumor antigens to the immune system of the patient occurs, which eventually results in the generation of an immune response mediated by T cells on the cancer cells of the patient.

Thus, in another aspect, the invention relates to an *in vitro* method for obtaining mature dendritic cells presenting at least one HPV E7 antigen comprising:

(i) contacting dendritic cells with a conjugate of the invention, a polynucleotide of the invention, a gene construct of the invention, a vector of the invention, a cell of the invention, a composition of the invention or a pharmaceutical composition of the invention in conditions suitable for the maturation of the dendritic cells to take place and
(ii) recovering the mature dendritic cells.

Dendritic cells (DCs) are the most potent antigen-presenting cells (APCs) and have a unique capacity to interact with the non-activated T lymphocytes (naive T lymphocytes) and initiate the primary immune response, activating the CD4+ helper T lymphocytes and the CD8+ cytotoxic T lymphocytes.

In the absence of inflammation and of immune response, dendritic cells patrol through the blood, peripheral tissues, lymph and secondary lymphoid organs. In the peripheral tissues, dendritic cells capture self and foreign antigens. The antigens captured are processed giving rise to fragments thereof which pass to class I and II MHC molecules (for the activation of CD8+ or CD4+ T lymphocytes, respectively). This process of antigen capture, degradation and load is called antigen presentation. However, in the absence of stimulation, the peripheral dendritic cells present the antigens inefficiently. The exogenous signal or signals coming from the pathogens or the endogenous signal or signals induce the dendritic cells so that they initiate a development process called maturation, which transforms the dendritic cells into APCs and into T lymphocyte activators.

There are different types of dendritic cells which can be used in the invention. On one hand there are myeloid dendritic cells (mDCs) which are similar to the monocytes and which in turn are divided into two subtypes: mDC-1, which are the most common and which are the main stimulators of the cells T and mDC-2, which are rarer and the main function of which is to fight against wound infections.

On the other hand, there are plasmacytoid dendritic cells (pDCs), which are similar to plasma cells but which have certain features typical of myeloid dendritic cells.

Immature dendritic cells are derived from bone marrow hematopoietic stem cells. These stem cells differentiate into immature cells having a high endocytic capacity and low capacity to activate T cells. These cells have in their membrane different membrane receptors such as TLRs.

The bacterial and viral products, as well as inflammatory cytokines and other typical molecules, induce the maturation of the dendritic cells by means of direct interaction with the surface receptors of innate dendritic cells. T lymphocytes, through CD40-dependent and independent pathways, and endothelial cells contribute to the final maturation of dendritic cells by means of direct cell-to-cell contact and by means of cytokine secretion. Soon after a danger signal arises, the efficiency of the antigen capture, the intracellular transport and the degradation, and the intracellular MHC

molecule traffic are modified. The peptide load, as well as the half-life and the transfer to the cell surface of the molecules MHC are increased. The expression in the surface of the T cell co-stimulating molecules also increases. Dendritic cells thus become the most potent APCs, and the only ones capable of activating the non-activated T lymphocytes and initiating the immune response. Together with the modification of the capacities thereof in antigen presentation, the maturation also induces the massive migration of dendritic cells out of the peripheral tissues. The modifications in the expression of chemokine receptors and adhesion molecules, as well as the important changes in the cytoskeleton organization, contribute to the migration of dendritic cells through the lymph to the secondary lymph organs.

Dendritic cells respond to two types of signals: to the direct recognition of the pathogens (by means of receptors with a specific recognition pattern) and to the indirect recognition of the infection (by means of inflammatory cytokines, internal cell compounds and specific immune responses). In response to these signals, dendritic cells are activated and initiate their maturation process, which transforms them into efficient T cell stimulators. One of the most efficient signals for the maturation of DCs is mediated by the interactions of the toll-like receptors, TLRs, (TLR1-9) with their respective ligands (reviewed by Kaisho and Akira, Biochimica et Biophysica Acta, 2002, 1589: 1-13).

The immature dendritic cells used in the present invention can be primary culture cells. Thus, the dendritic cells used in the method of the invention can be autologous or heterologous.

As used herein, the term "autologous" means that the cells are from the same individual.

As used herein, the term "heterologous" means that the cells are from a different individual.

Dendritic cells can be generated *in vitro* from peripheral blood mononuclear cells (PBMCs) using a protocol which would basically consist of seeding PBMCs in a culture bottle such that the adhesion of said cells is allowed. After that the cells would be treated with interleukin 4 (IL4) and granulocyte-macrophage colony-stimulating factor (GM-CSF) leading to the differentiation of the cells into immature dendritic cells (iDCs) in approximately one week. Optionally, the cells can be maturated treating them with tumor necrosis factor alpha (TNF$\alpha$).

Dendritic cells can be obtained using standard methods from suitable sources. These tissues suitable for the isolation of dendritic cells include the peripheral blood, spinal cord, tumor-infiltrating cells, peritumor tissue-infiltrating cells, biopsies of lymph nodes, thymus, spleen, skin, umbilical cord blood, monocytes obtained from peripheral blood, CD34- or CD14-positive cells obtained from peripheral blood, as well as any other suitable tissue or fluid.

Optionally, stable cell cultures can be used. Document WO9630030 describes methods for obtaining dendritic-like cell/tumor cell hybridomas and pluralities of dendritic-like cell/tumor cell hybrids. These hybrids and hybridomas are generated for the fusion of tumor cells with dendritic-like cells. For example, immortal tumor cells from an autologous tumor cell line can be fused with autologous HLA-matched allogeneic dendritic-like cells. The autologous tumor cell lines can be obtained from primary tumors and from their metastases. Alternatively, immortal dendritic-like cells of a autologous or allogeneic HLA-matched dendritic-like cell line can be fused with autologous tumor cells. Document WO/2002/048167 also describes methods for generating stable dendritic cell lines. Another cell line that can be used is CB1 (Paglia P. et al. 1993. Journal of Experimental Medicine, Vol 178, 1893-1901).

A first step of the method of the invention consists of contacting dendritic cells with a conjugate of the invention, a polynucleotide of the invention, a vector of the invention, a gene construct of the invention, a cell of the invention, a composition of the invention or a pharmaceutical composition of the invention in conditions suitable for the maturation of the dendritic cells to take place.

The invention contemplates any possible way of contacting the dendritic cells with a conjugate of the invention, a polynucleotide of the invention, a vector of the invention, a gene construct of the invention, a cell of the invention, a composition of the invention or a pharmaceutical composition of the invention. The person skilled in the art will appreciate that, depending on the type of compound, the contacting is carried out differently. Thus, in the event that it is the conjugate of the invention or a peptide of the invention, these can be directly added to the culture medium in which are the cells are located or can be bound to a surface of plastic, glass, etc. which will be exposed to the dendritic cells. Forms to bind the components of the conjugate of the invention as well as the peptide of the invention to solid surfaces are known by a person skilled in the art.

In the event that it is a gene construct of the invention or a vector of the invention, the techniques used to introduce said components in a cell (cell of the invention) have been previously described in the section of gene constructs of the invention. In a particular embodiment, the cells of the invention have in their membrane the components of the conjugate of the invention such that they are accessible to the dendritic cells.

"Conditions suitable for the maturation to take place", is understood as all those culture conditions (oxygen, temperature, humidity, nutrients etc) which allow activating the dendritic cells, after being contacted with a conjugate of the invention, a polynucleotide of the invention, a vector of the invention, a cell of the invention, a composition of the invention or a pharmaceutical composition of the invention, such that at least one HPV E7-derived antigen has been presented. This activation occurs when the immature dendritic cells have phagocytized some of the presented antigens and have degraded said antigens into small pieces, presenting said parts in their surface by using histocompatibility system molecules (MCH). The mature dendritic cells simultaneously upregulate membrane receptors acting as co-receptors in the activation of the T cells, such as CD80 (B7.1), CD86 (B7.2), and CD40, such that their capacity to

activate said T cells is thus increased. The mature dendritic cells also upregulate the expression of CCR7, a receptor which induces the travel of the dendritic cells throughout the blood stream to the spleen and from there their passage to the lymph system. The mature dendritic cells are capable of activating helper T cells, killer T cells and B lymphocytes presenting the antigens which they have processed.

Thus, mature dendritic cell presenting at least one HPV E7 antigen is understood as that dendritic cell which, after capturing am HPV E7 antigen, is capable of presenting said antigen in the surface of its membranes bound to the major histocompatibility complex (MHC) after having processed it. Additionally, the mature dendritic cells can present the above indicated upexpressed membrane receptors.

In another step, the cells are maintained under conditions suitable for the internalization, processing and presentation of one or more peptides of derivatives of the conjugate of the invention. The conditions suitable for the internalization, processing and presentation of the at least one antigenic peptide derived from the conjugate of the invention can be determined using standard assays for determining the activation of dendritic cells.

The maturation of the DCs can be followed using a number of molecular markers and of phenotypic alterations of the cell surface. These changes can be analyzed, for example, using flow cytometry techniques. The maturation markers are typically marked using specific antibodies and the DCs expressing a marker or a group of markers can be separated from the total of DCs using, for example, FACS cell sorting. The DC maturation markers include genes which appear expressed at high levels in mature DCs compared with immature DCs. These markers include, MHC class II antigens of the cell surface (in particular HLA-DR), co-stimulating molecules such as CD40, CD80, CD86, CD83, cell traffic molecules such as CD45, CD11c and CD18, etc. Furthermore, the maturation of DCs can be determined measuring the expression of certain Notch ligands such as the Delta-like ligand (DLL4), Jagged1 and Jagged2 which are associated with the induction of the Th1 response. On the other hand, mature dendritic cells can be identified using their ability to stimulate the proliferation of allogeneic T cells in a mixed lymphocyte reaction (MLR). Furthermore, it has been generally seen that while immature DCs are very efficient in the internalization of antigens but have a low antigen presentation, mature DC cells have a low internalization of antigens but are very efficient in presenting antigens.

The antigen-presenting function of the dendritic cells can be measured using MHC-limited, antigen-dependent T cell activation assays as well as other assays which are well known for the persons skilled in the art such as the capacity for *in vitro* stimulation in peripheral blood lymphocytes, for example, determining the amount of IFN-$\gamma$ produced by CD8+ lymphocytes in the presence of DCs. This determination can be carried out using the technique called ELISPOT. The activation of T cells can additionally be determined measuring for example the induction of cytokine production by the stimulated dendritic cells. The stimulation of the cytokine production can be determined using a large variety of standard techniques, such as ELISA, which are well-known by a person skilled in the art.

Other cytotoxicity assays such as the binding of target cells with tritiated thymidine (3H-TdR) can be used. 3H-TdR is incorporated in the nucleus of the cells. The release of·H-TdR is a measure of cell death due to DNA fragmentation.

In a second step of the method of the invention the mature dendritic cells obtained in step (a) are recovered.

Different strategies can be used to recover the mature cells obtained in step a) of the method of the invention. For example, the membrane markers expressed by mature cells and which have been previously described, such as for example CD80, can be used.

The expression of cell surface markers can be determined, for example, by means of flow cytometry using conventional methods and apparatuses. For example, the Becton Dickinson Calibur FACS (fluorescent-activated cell sorting) system using commercially available antibodies and usual protocols known in the art can be used. Thus, the cells presenting a signal for a specific cell surface marker in the flow cytometry above the background signal can be selected. The background signal is defined as the signal intensity given by a non-specific antibody of the same isotype as the specific antibody used to detect each surface marker in the conventional FACS analysis. In order for a marker to be considered positive, the observed specific signal has to be more than 20%, preferably, 30%, 40%, 50%, 60%, 70%, 80%, 90%, 500%, 1000%, 5000%, 10000% or above, intense in relation to the intensity of the background signal using conventional methods and apparatuses (for example, a Becton Dickinson Calibur FACS system used with commercially available antibodies and usual protocols known in the art).

The dendritic cells obtained by means of the method according to the invention have proved to be useful for the treatment of diseases which respond to the generation of an immune response against E7 antigens. Using said method, mature cells presenting at least one antigen from the conjugate of the invention are obtained. Thus in another aspect, the invention relates to antigen-presenting dendritic cells presenting at least one antigen from the conjugate of the invention and which are CD40-positive obtained by means of the method of the invention or dendritic cells of the invention.

In another aspect, the invention relates to dendritic cells of the invention, for their use in medicine. Said dendritic cells of the invention can be used to cause an immune response in a patient using the latter as a DC vaccination, i.e. by means of the administration to said patient of said cells. Thus in another aspect, the invention relates to a dendritic cell of the invention for the generation of an immune response in a patient. In other words, the invention relates to a method for causing an immune reaction in a subject which comprises the administration to a subject of the antigen-presenting cell.

The vaccination with DC is carried out by administering the antigen-presenting DC to a subject (for example a human patient) in whom an immune response is induced. The immune response typically includes a CTL response against target cells which are marked with the antigenic peptides (for example the components of the conjugate of the invention). These target cells are typically cancer cells. When the modified DCs are to be administered to a patient, these cells are preferably isolated from stem cells of the same patient (i.e., DCs are administered to an autologous patient). However, the cells can be administered to allogeneic patients who are compatible with respect to the HLA or to allogeneic patients where there is no coincidence. In this latter case immunosuppressive drugs must be administered to the patient receiving the cells.

The cells can be administered in any suitable form, preferably with a carrier (for example saline solution). The administration will normally be intravenous, but intra-articular, intramuscular, intradermal, intraperitoneal or subcutaneous administrations are also acceptable. The administration or immunization can be repeated at different time intervals.

The DC injection can be combined with the administration of cytokines which act such that the number of DCs and their activity is maintained such as GM_CSF, IL-12.

The dose administered to a patient must be efficient to induce an immune response which can be detected using assays which measure the proliferation of T cells, the cytotoxicity of T lymphocytes and/or the beneficial therapeutic effect of response of the patients over time. $10^6$ to $10^9$ DC cells are typically injected, provided that they are available. The vaccines can be administered one or more times to a patient to achieve beneficial results. The time between the first and the successive dose or doses of the vaccine depend on a variety of factors, which include the health of the patient, age, weight, etc. The vaccine can be administered at any suitable time interval, for example including but without being limited to, once a week, once a day every two weeks, once a day every three weeks, once a month. In a particular embodiment, the vaccine can be administered indefinitely. In another particular embodiment, the vaccine is administered three times in intervals of two weeks. The doses of the vaccine also depend on a variety of factors, which include but are not limited to the health of the patient, stability, age, weight, etc. Once a sufficient immunity level involving a clinical benefit has been achieved, booster doses can be used, which are generally administered with a lower frequency (for example monthly or half-yearly doses).

The DCs used in the method for causing an immune response are preferably formulated so that they can be used as an off-the-shelf drug or ready for their use in the event that there is histocompatibility between the cells of the preparation and those of the treated patient. The lack of compatibility between the subject object of the therapy and the dendritic cells can result in a reduction of the effect of the vaccine, either because there is a premature elimination of the cells (especially after multiple administrations) or due to the generation of a strong anti-allotypic response distracting the immune system from the intended target. In this context, the use of vaccines in which at least some of the HLA Class I alleles in the dendritic cells of the invention (especially in the locus A and more particularly in the A2 allele) are shared with the patient is advantageous. Thus at least some of the tumor antigens will be presented in autologous class I molecules, whereby the antitumor response will be increased and the anti-allotypic response will be reduced.

A partial coincidence can be obtained using a vaccine with DCs made with cells having two or more of the most common HLA-A allotypes (HLA-A2, A1, A19, A3, A9, and A24). A total coincidence for most of the patients can be achieved by providing the physician with a set of different DCs from where different possibilities having only an allotype in the locus HLA-A can be selected. The treatment will involve the identification of one or more HLA allotypes in the patient using standard tissue-taping methods, and the treatment of the patients with the DCs having the HLA allotype or allotypes which coincide with those of the patient. For example a patient who is HLA-A2 and HLA-A19 can be treated either with cells homozygous for HLA-A2 or for HLA-A19 or with a mixture of both.

Potential negative effects of the lack of coincidence of HLA can also be treated generating immunotolerance against the foreign allotypes. During the preparation of the vaccine the DC cells are divided into two groups: a group for generating tolerogenic immature cells and the other group for generating mature DCs for the antigen presentation. The tolerogenic cells are designed such that they generate an acceptance of the mature cells. The tolerogenic cells will be administered one or more times to the patient so that a sufficient degree of absence of immune response (measured for example in a mixed lymphocyte reaction) is generated. Once the patient is tolerant (after one week or one month) the mature antigen-presenting cells are administered to the subject in the amount and in the frequency which is necessary for an immune response against the target tumor antigen.

In a particular embodiment, the antigen-presenting dendritic cells of the invention are autologous to the subject to be treated. The composition of the vaccine can include an adjuvant. The adjuvant can be any available adjuvant or a combination thereof. Examples of adjuvants have been mentioned in the section of medical uses of the conjugates and compositions of the invention.

In another aspect, the invention relates to the use of dendritic cells of the invention for the manufacture of a medicament for the prevention and the treatment of an infection caused by the human papilloma virus and/or of cervical cancer associated with HPV infections. Alternatively, the invention relates to dendritic cells of the invention for the prevention and the treatment of an infection caused by the human papilloma virus and/or of cervical cancer associated

with HPV infections. Finally, the invention relates to a method for the treatment of an infection caused by the human papilloma virus and/or of cervical cancer associated with HPV infections in a subject which comprises the administration to said subject of dendritic cells according to the invention.

The invention is described below by means of the following examples which must be considered as merely illustrative.

EXAMPLES

MATERIALS AND METHODS

Mice and cell lines

Specific pathogen-free five-week-old female C57BL/6 mice were purchased from Harlan Laboratories (Barcelona, Spain) and kept in the CIMA animal facilities under pathogen-free conditions with water and food *ad libitum*. Experiments involving animals were conducted according to the institutional guidelines for animal care.

THP1 cells (American Type Culture Collection ATCC, Manassas, VA) were used for *in vitro* assays of monocyte activation by EDA derived fusion proteins. TC-1, tumor cells expressing HPV16-E6 and HPV16-E7 proteins derived from primary mouse lung epithelial cells, were obtained from the American Type Culture Collection (LGC Promochem, Molsheim, France). Cells were maintained in RPMI 1640 with GlutaMAX supplemented with 10% heat-inactivated fetal calf serum, 100 units/ml penicillin, 100μg/ml streptomycin, 0.4 mg/ml geneticin and 5 x 10-5 mol/l 2-mercaptoethanol (Life Technologies, Cergy-Pontoise, France). 5 x 105 TC-1 cells were injected into the shaved back (left side) of C57BL/6 mice in 200 μL PBS. Tumor size, presented as the average of two perpendicular diameters (millimeters), was measured at regular intervals. EL-4 (mice timoma) tumor cells were obtained from the American Type Culture Collection (LGC Promochem, Molsheim, France) and were used as target cells for cytotoxicity studies (were incubated with or without DTc peptides) and as tumor negative controls since they do not express any HPV protein.

Reagents

The synthetic $E7_{49\text{-}57}$ peptide [RAHYNIVTF (SEQ ID NO: 67) one-letter code for amino acid] corresponding to the HPV16-E7 H2-Db-restricted epitope was purchased from Proimmune (Oxford, UK). Polyinosinic-polycytidylic acid (PIC, poliI:C, pIC o pI:C) was purchased from Invivogen (San Diego, CA) and was diluted in PBS before injection. CpG phosphorothioate oligodeoxynucleotides Type B (CpG 1826: 5'-TCCATGACGTTCCTGACGTT-3') were synthesized by Proligo (Paris, France). Thirty μg of CpG oligodeoxynucleotides were diluted in 50 μL Optimen medium (Gibco, Grand Island, NY, USA) and mixed with 60 μg DOTAP (Roche, Mannheim, Germany) diluted in 100 μL Optimen. Cyclophosphamide (CPA) (Sigma, Steinheim, Germany) was diluted in PBS before injection and administered 24 hours before vaccination. The various antigenic formulations and adjuvants were injected simultaneously. Intravenous administration was performed by retroorbital injection in a volume of 200 μL.

Preparation of the recombinant EDA-HPVE7 fusion protein

The plasmid pET20b-EDA, expressing the extra domain A from fibronectin was prepared as previously described (Lasarte, Casares et *al.* 2007) and was used for the construct of the expression plasmid pET20b-EDA-HPVE7 to express a fusion protein containing the 1-29 first amino acids from HPV-E7 protein linked to the N-terminus of EDA protein and amino acids 43-98 amino acids from HPV-E7 to the C-terminus of EDA. This expression plasmid was constructed in two steps as described below. RNA from TC-1 cells was isolated from $1x10^7$ cells according to the methods of Chomczynski and Sacchi (Chomczynski and Sacchi 1987) using Ultraspec (Biotecx, Houston, TX, USA) according to manufacturer's instructions. Total RNA was reverse transcribed and the gene coding for the HPV-E7 protein was amplified by PCR as described previously (Lasarte, Casares et *al.* 2007) using the upstream first UP-1 CATATGCATGGAGATACACCTAC [SEQ ID NO: 68] (containing the NdeI restriction site, underlined) and the downstream first DW-1 GCGGCCGCTGGTTTCTGAGAACAGAT [SEQ ID NO: 69] (containing the NotI restriction site) . The resulting PCR products were cloned inpCR2.1-TOPO using the TOPO TA cloning kit (Invitrogen, Carlsbad, CA, USA), leading the plasmid pCR2.1-TOPO-HPV-E7. For the firs step on the construct of the expression plasmid pET20b-EDA-HPVE7, a PCR reaction using the primers UP-1 (SEQ ID NO: 68) and DW-2 (CATATGATTTAATTGCTCATAACA, SEQ ID NO: 70) and the plasmid pCR2.1-TOPOHPV-E7 as template. The resulting fragment was subcloned in pCR2.1-TOPO leading the plasmid pCR2.1-TOPO-(1-29)E7. This plasmid was digested with NdeI restriction enzyme and the resulting fragment was subcloned in the NdeI digested pET20b-EDA plasmid leading the plasmid pET20b-EDA-(1-29)E7. In a second step, a PCR reaction using the plasmids UP-2 (GCGGCCGCAGGACAAGCAGAACCGGA, SEQ ID NO: 71) and DW-1 (SEQ ID NO: 69) and plasmid pCR2.1-TOPO-HPV-E7 as template was carried out. The resulting PCR product was subcloned

in pCR2.1-TOPO leading the plasmid pCR2.1-TOPO (43-98)E7, which was digested with NotI to purify the fragment coding for this part of E7 protein. This product was subcloned in the plasmid pET20b-EDA-(1-29)E7 previously digested with NotI. After this process, we obtained the plasmid pET20b-EDA-HPVE7 which was sequenced to confirm the correct expression of the fusion protein (1-29)E7-EDA-(43-98)E7 carrying six histidine residues (6xHis tags) at the carboxy terminus. This plasmid pET20b2-26 was transfected into BL21(DE3) cells for the expression of the recombinant protein which was purified from the soluble fraction of cell extracts by affinity chromatography (Histrap, Pharmacia) by using an FPLC platform (AKTA, Pharmacia). The eluted protein was desalted using Hitrap desalting columns (Pharmacia), and concentrated using Amicon Ultra 4-5000 MWCO Centrifugal filter device (Millipore Carrighwahill, Ireland). The recombinant protein was purified from endotoxins by using Endotrap columns (Profos Ag, Regensburg, Germany) until the levels of endotoxin were below 0.2 EU/μg protein as tested by Quantitative Chromogenic Limulus Amebocyte Lysate assay (Cambrex, Walkersville, MD, USA).Purified recombinant protein was separated by SDS-PAGE and stained with Coomassie blue using the Bio-Safe Coomassie reagent (Bio-Rad, Hercules, CA) according to manufacturer's instructions (Figure 1).

Protein characterization by western blotting and by Mass Spectrometry.

Western Blot Analysis: Purified proteins were loaded onto 10% SDS-PAGE gels followed by electrophoretic transfer to nitrocellulose membranes. Histidine tagged proteins were detected using anti-His antibodies (Qiagen) and secondary antimouse horseradish peroxidase-conjugated antibody (Amersham Pharmacia). Protein bands were detected by enhanced chemiluminescence (Amersham Pharmacia Biotech, Freiburg, Germany). Peptide mass fingerprints (PMF) were obtained from the tryptic digests using a MALDI-TOF GL-REF mass spectrometer from Waters. Briefly, 1.2 μL of the tryptic digest were mixed with an equal volume of α-cyano-4-hydroxy-trans-cynnamic acid in 0.1% TFA in 50% acetonitrile and spotted onto a MALDI-TOF target plate. Data processing was performed with Masslynx 4.0 (Waters) using ACTH as internal lock mass standard.

Microcapillary reversed phase LC was performed with a CapLC (Waters) capillary system. Reversed-phase separation of tryptic digests was performed with an Atlantis, C18, 3 μm, 75 μm × 10 cm Nano Ease fused silica capillary column (Waters) equilibrated in 5% acetonitrile, 0.2% formic acid. After injection of 6 μL of sample, the column was washed during 5 min with the same buffer and the peptides were eluted using a linear gradient of 5-50% acetonitrile in 30 min at a constant flow rate of 0.2 μL/min. The column was coupled online to a Q-TOF Micro (Waters) using a PicoTip nanospray ionization source (Waters). The heated capillary temperature was 80°C and the spray voltage was 1.8-2.2 kV. MS/MS data were collected in an automated data dependent mode. The three most intense ions in each survey scan were sequentially fragmented by collision induced dissociation (CID) using an isolation width of 2.5 and a relative collision energy of 35%. Data processing was performed with Masslynx 4.0. Protein identifications based on PMF were only accepted when the Masslynx score was at least 7 and the matched peptides represent at least 30% of the proposed protein sequence. Protein identifications from MS/MS data were only considered for score values above 7 and when were based on the sequence of at least 3 independent peptides.

*In vitro* analysis of monocyte activation. THP-1

THP-1 cells were plated at 1 x $10^6$ cells/well and cultured overnight at 37°C and 5% $CO_2$ in complete medium for stabilization of the culture. Different concentrations of the indicated antigens were added to the cultures and after 15 hour of incubation, culture supernatants were harvested. The concentration of human TNF-α released to the medium by the THP-1 cell line was quantified using a commercial ELISA assay (BD Pharmingen), according to manufacturer's instructions.

*In vitro* analysis of DC maturation

Bone marrow (BM)-derived dendritic cells were generated from mouse femur marrow cell cultures. After lysing erythrocytes with ACK lysing buffer, bone marrow cell were washed and subsequently depleted of lymphocytes and granulocytes by incubation with a mixture of antibodies against CD4 (GK1; ATCC, Manassas, VA), CD8 (53.6.72; ATCC), Ly-6G/Gr1 (BD-Pharmingen; San Diego, CA) and CD45R/B220 (BD-Pharmingen) followed by rabbit complement. Remaining cells were grown at 106 cells/ml in 12-well plates in CM (RPMI 1640 supplemented with 10% FCS, 2 mM glutamine, 100 U/ml penicillin, 100 μg/ml streptomycin and 5x$10^{-5}$ M 2-mercaptoethanol) supplemented with 20 ng/ml of mGM-CSF and 20 ng/ml of mIL-4 (both from Peprotech; London, UK). Every two days, two thirds of medium was replaced with fresh medium containing cytokines. Non-adherent dendritic cells were harvested at day 7 and cultured in the presence or absence of different stimuli at 37°C and 5% $CO_2$. After 24 h of culture, supernatants were harvested and IL-12 (p70) and TNF-α were measured by ELISA (BD-Pharmingen), according to manufacturer's instructions. Expression of DC maturation markers was measured by flow cytometry. Cells were pre-incubated with a rat anti-CD16/32

mAb (2.4G2 clone, BD Pharmingen) for 15 min, to block non-specific binding of primary antibodies. After this initial incubation, cells were stained with the primary antibodies at 4°C for 15 min, washed and acquired on FACSscan cytometer (BD Biosciences, San Diego, CA) and analyzed using Cell Quest software (BD Biosciences). The antibodies used were: anti-IAβb (25-9-17 clone), anti-H-2Kb (AF6-88.5 clone), anti-CD40 (3/23 clone), anti-CD54 (3E2 clone), anti-CD80 (16-10A1clone), anti-CD86 (GL1 clone), anti-CD11c (HL-3 clone), all from BD Pharmingen.

Statistical analysis

Monolix software (http://www.monolix.org/) was used for analysis of tumor growth data with non-linear mixed effect models. Mean diameters of tumors over time were fitted using the model described in equation 1.1 and treatments were compared using the likelihood ratio test.

$$y_{ij} = y_0 + k_c \cdot t_{ij} - k_a \cdot e^{-k_e \cdot t_{ij}}$$

where $Y_{ij}$ is the tumor diameter in mouse i at time $t_{ij}$, $y_0$, $k_c$, $k_a$ and $k_e$ are the coefficients of the model that express, respectively, the initial tumor diameter, the tumor growth rate, the intrinsic killing activity of effector cells and the elimination rate of effector cells.

Kaplan-Meier plots were used to analyze survival. Prism software (GraphPad Software, Inc. San Diego, USA) was used to determine the significance of differences in survival curves with a log rank test. The results of the *in vivo* killing analysis were analyzed by ANOVA followed by Dunnett's Multiple Comparison test. p values less than 0.05 were considered to be statistically significant.

EXAMPLE 1

EDA-HPVE7 fusion protein is able to induce maturation of bone marrow-derived dendritic cells *in vitro*

Document WO06134190 describes that the recombinant protein encompassing the extra domain A from fibronectin (EDA), an endogenous ligand for Toll-like receptor 4 (TLR4), is able to bind to and activate TLR4 signaling pathway, as has been described. EDA also stimulated the production by DC of proinflammatory cytokines, such as IL-12 or TNF-alpha, and induced their maturation *in vitro* and *in vivo.* Therefore, it was assayed if the recombinant protein EDA-HPV-E7 was also able to induce maturation of BMDC *in vitro.* It was found that incubation of BMDC with 500 nM of EDA-HPV-E7 was able to upregulate expression of the maturation markers IAb, CD54 and, in a lesser extend, CD86 molecules (Figure 2). Moreover, EDA-HPV-E7 was able to strongly stimulate the production of IL-12 cytokine by BMDC (Figure 3A). Similarly, the recombinant fusion protein EDA-HPVE7 was able to induce the production of TNF-α cytokine by the human monocyte cell line THP-1 (Figure 3B) which also expresses TLR4 molecule. These results indicate that the fusion protein between EDA and HPVE7 protein maintains the proinflammatory properties of EDA.

EXAMPLE 2

EDA-HPVE7 fusion protein induces HPVE7 specific CTL *in vivo* in the absence of coadjuvants.

It was assayed if mice immunized with the EDA-HPVE7 fusion protein developed HPVE7-specific T cell responses. The capacity of the fusion protein was compared with the synthetic E7(49-57) peptide comprising the cytotoxic T cell determinant recognized by C57BL6 mice. Mice were immunized i.v with 2 nmol EDAHPV-E7 or with peptide DTc/E7(49-57) in PBS. Seven days after immunization, mice were sacrificed and spleen cells were cultured in the presence or absence of peptide DTc for 5 days. CTL activity was measured using a conventional chromium release assay using radio labeled EL4 cells pulsed with DTc peptide or without peptide as target cells. (Figure 4A) (On the graphic, the absolute values are shown, said values are calculated subtracting the value of lysis obtained without peptide from the value obtained in the presence of peptide).It is shown that mice immunized with EDA-HPVE7 protein have higher levels of CTL activity against target cells pulsed with the cytotoxic T cell epitope E7. It was also measured by means of ELISPOT the number of IFN-γ producing cells cultures in the presence or absence of peptide E7(49-57) or in response to irradiated TC1 tumor cells (which expresses HPVE7 protein) or in response to tumoral cells EL-4 (negative control) in splenocytes from mice immunized with EDAHPV-E7 or with E7(49-57) peptide (Figure 4B). It was found that those mice immunized with EDA-HPVE7 have a higher number of IFN-γ producing spots specific for E7(49-57) or TC1 cells, confirming the higher immunogenicity of this protein in comparison with the cytotoxic T cell determinant alone.

EXAMPLE 3

EDA-HOVE7 fusion protein induces HPVE7 specific CTL *in vivo in presence of* coadjuvants.

It was assayed if mice immunized with the EDA-HPVE7 fusion protein developed improved HPVE7-specific T cell responses in the presence of the TLR3 ligand poliI:C (pI:C). Mice were immunized i.v. with 2nmol EDA-HPVE7 or DTc/E7 (49-57) peptide in the presence of pI:C (50 μg/mice). Seven days after immunization, mice were sacrified and spleen cells were cultured in the presence or absence of DTc/E7(49-57) peptide. After five days of culture, the CTL activity was measured using a conventional chromium release assay using as target cells radio labeled EL4 cells pulsed with or without DTc peptide (Figure 5A) or irradiated TC-1 cells (Figure 5B) (the graph shows the absolute values which are calculated subtracting the value of lysis obtained without peptide from the value obtained in the presence of peptide). In parallel, the splenocytes from mice immunized with EDAHPV-E7 or with DTc/E7 (49-57) peptide were incubated during 24 hours in the presence or absence of the E7(49-57) peptide or TC1 cells or irradiated EL-4 cells, for measuring using ELISPOT the IFN-γ production (Figure 5C).

EXAMPLE 4

Therapeutic Efficacy of EDA-HPVE7 fusion protein combined with poly I:C and cyclophosphamide.

To better evaluate the potency of EDA-HPVE7 as an antigen delivery system and as a vaccine, the therapeutic efficacy of this fusion protein was compared with that of the E7(49-57) peptide, both supplemented with TLR ligand pI:C. C57BL/6 6 Mice were therefore injected s.c. with 5 x $10^5$ TC-1 cells and treated therapeutically 25 days later, when tumor mean diameters were approximately 8 mm. Thus, mice were treated i.v. with the following combinations of EDA vaccines: (i) 2 nmol of EDA-HPVE7 plus 50 μg of pI:C; (ii) 2 nmol of peptide E7(49-57) plus 50 μg of pI:C; (iii) 2 nmol of the E7(49-57) peptide plus 2nmol EDA plus 50 μg of pI:C;(iv) 2 nmol of EDA plus 50 μg of pI:C; (v) 50 μg of pI:C alone; (vi) 2 nmol of EDAHPVE7 alone or; (vii) with PBS.

In mice injected with PBS (control group), tumors grew progressively and mice had to be sacrificed between days 30 and 40 (Figure 6A). Most of the tumors treated with adjuvants without antigen, specifically pI:C and EDA+pI:C treated mice, followed the same tumor growth kinetics although in some mice, a delay in tumor growth was observed. This delay led to a slight, statistically non-significant prolongation of survival (Figure 6B). Addition of E7 peptide (E7(49-57)) to the immunization cocktail improved slightly the tumor growth arrest induced by adjuvants (Figure 6A). Some tumors showed a tumor size remission for several days and even in a mouse, the tumor disappeared completely, however, tumors continued growing and mice were finally sacrificed. The fusion protein EDAHPVE7 alone was unable to eradicate the tumors, although a delay in tumor growth was observed. However, combination of EDA-HPVE7 with pI:C, a mild adjuvant that binds to TLR3, was able to achieve eradication of these large, established tumors in 60% of mice (Figure 6A). Both tumor growth (Figure 6A) and survival (Figure 6B) differed significantly from pI:C-treated mice ($p<0.0004$, determined using the likelihood ratio test between the mice treated with E7(49-57) + pI:C vs. EDA-HPVE7 + pI:C (Figure 6A), and $p<0.05$ determined using the long-rank test for the comparison of the survival curves (Figure 6B)). Therefore, both covalent binding of antigen and EDA and adjuvant coadministration are required to eradicate large tumors in mice.

The therapeutic efficacy of EDA-HPV based vaccination in a more difficult situation was also assayed. C57BL/6 mice were therefore injected s.c. with 5 x $10^5$ TC-1 cells and treated therapeutically 35 days later, when tumor mean diameters were approximately 12-15 mm. In this model EDA-HPVE7 was combined with the treatment of mice with a low dose of the chemotherapeutic agent cyclophosphamide (CPA, 175 mg/kg). Twenty four hours later, mice were immunized with EDA-HPV-E7 (2 nmol/mice) and CpG-B/DOTAP (30 μg/mice). Mice immunized with PBS alone or with the adjuvants and CPA treatment alone (Figure 7B) were used as controls. All animals received a second dose from the corresponding treatment at day 50 from the study as it is indicated in the diagram represented in figure 7A.

It was found that treatment of mice with two immunizations with the combination of EDA-HPVE7 plus CpG/DOTAP and CPA was able to reject these very big tumors in 50% of mice, whereas only 12% of mice were cured after treatment with the adjuvants and the CPA administration ($p=0.0064$, determined using the likelihood ratio test between the CPA+CpG/DOTAP vs. CPA+CpG/DOTAP+EDA-HPVE7 treated mice (Figure 7A), and $p<0.05$ determined using the log-rank test for the comparison of survival curves) (Figura 7C).

In summary, the recombinant fusion protein EDA-HPVE7 has been proved to be able to induce maturation of dendritic cells, induce the activation of *in vivo* anti-tumor immune response and able to eradicate large, and well established tumors expressing HPVE7 protein. These data suggest that EDA-HPVE7 protein may be considered for the development of an alternative therapy against human cervical carcinoma.

## Claims

**1.** A conjugate comprising:

i) the fibronectin EDA region or a functionally equivalent variant thereof showing a degree of identity with respect to the fibronectin EDA region greater than at least 70% and
ii) at least one HPV E7-derived antigenic peptide, wherein the at least one HPV E7-derived antigenic peptide is a fragment of the HPV E7 protein which is capable of stimulating the immune system of a mammal such that an immune response against said protein capable of inhibiting the growth of tumors caused by the expression of E7 or of inhibiting the proliferation of HPV is generated,

wherein components (i) and (ii) are covalently coupled and wherein the conjugate promotes a cytotoxic response towards the antigenic peptide or peptides.

**2.** The conjugate according to claim 1, wherein component (ii) comprises an antigenic peptide containing amino acids 1 to 29 of SEQ ID NO: 51, an antigenic peptide containing amino acids 43 to 98 of SEQ ID NO: 52 or both.

**3.** The conjugate according to any of claims 1 or 2, wherein component (ii) forms a single polypeptide chain with component (i).

**4.** The conjugate according to any claims 1 to 3, wherein the EDA region is flanked by two HPV E7 antigenic peptides.

**5.** The conjugate according to claim 4, comprising the sequence SEQ ID NO: 53 or SEQ ID NO: 72.

**6.** A polynucleotide or gene construct encoding a conjugate according to any of claims 3 to 5.

**7.** A cell containing a polynucleotide or a gene construct according to claim 6.

**8.** A composition comprising, together or separately:

(i) a conjugate according to any of claims 1 to 5, a polynucleotide or gene construct according to claim 6, or a host cell according to claim 7 and
(ii) a TLR ligand.

**9.** A composition comprising, together or separately:

(i) a conjugate according to any of claims 1 to 5, a polynucleotide or gene construct according to claim 6, or a host cell according to claim 7,
(ii) a TLR ligand and
(iii) a chemotherapeutic agent.

**10.** The composition according to claims 8 or 9, wherein the TLR ligand is selected from the group consisting of a TLR3 ligand, a TLR9 ligand and a combination of both.

**11.** The composition according to claim 10, wherein the TLR3 ligand is poly(I:C), wherein the TLR9 ligand is an oligonucleotide comprising at least one CpG motif or wherein the chemotherapeutic agent (iii) is cyclophosphamide.

**12.** A pharmaceutical composition comprising a conjugate according to any of claims 1 to 5, a polynucleotide or gene construct according to claim 6, a cell according to claim 7 or a composition according to any of claims 8 to 11 and at least one pharmacologically acceptable carrier or adjuvant.

**13.** A conjugate according to any of claims 1 to 5, a polynucleotide or gene construct according to claim 6, a cell according to claim 7 or a composition according to any of claims 8 to 11 for its use in medicine.

**14.** Use of a conjugate according to any of claims 1 to 5, a polynucleotide or gene construct according to claim 6, a cell according to claim 7 or a composition according to any of claims 8 to 11 for the manufacture of a vaccine.

**15.** A conjugate according to any of claims 1 to 5, a polynucleotide or gene construct according to claim 6, a cell according to claim 7 or a composition according to any of claims 8 to 11 for use in the prevention or treatment of an infection caused by the human papilloma virus and/or of cervical cancer associated with HPV infections.

**16.** An in *vitro* method for obtaining mature dendritic cells presenting at least one HPV E7 antigen, comprising:

i) contacting dendritic cells with a conjugate according to any of claims 1 to 5, a polynucleotide or gene construct according to claim 6 a cell according to claim 7 or a composition according to any of claims 8 to 11 in conditions suitable for the maturation of the dendritic cells to take place and
ii) recovering the mature dendritic cells.

**Patentansprüche**

**1.** Konjugat, umfassend:

i) die Fibronektin-EDA-Region oder eine funktionell äquivalente Variante davon, die einen Grad an Identität in Bezug auf die Fibronektin-EDA-Region von größer als wenigstens 70% zeigt, und
ii) wenigstens ein HPV E7-abgeleitetes antigenes Peptid, wobei das wenigstens eine HPV E7-abgeleitete antigene Peptid ein Fragment des HPV E7-Proteins ist, welches dazu in der Lage ist, das Immunsystem eines Säugetiers zu stimulieren, so dass eine Immunantwort gegen das Protein erzeugt wird, die dazu in der Lage ist, das Wachstum von Tumoren, verursacht durch die Expression von E7, zu inhibieren oder die Proliferation von HPV zu inhibieren,

wobei die Komponenten (i) und (ii) kovalent gekoppelt sind und wobei das Konjugat eine zytotoxische Antwort auf das/die antigene/n Peptid oder Peptide fördert.

**2.** Konjugat gemäß Anspruch 1, wobei Komponente (ii) ein antigenes Peptid, welches die Aminosäuren 1 bis 29 von SEQ ID NO: 51 enthält, ein antigenes Peptid, welches die Aminosäuren 43 bis 98 von SEQ ID NO: 52 enthält, oder beide umfasst.

**3.** Konjugat gemäß irgendeinem der Ansprüche 1 oder 2, wobei Komponente (ii) eine einzelne Polypeptidkette mit Komponente (i) bildet.

**4.** Konjugat gemäß irgendeinem der Ansprüche 1 bis 3, wobei die EDA-Region durch zwei HPV E7-antigene Peptide flankiert wird.

**5.** Konjugat gemäß Anspruch 4, welches die Sequenz SEQ ID NO: 53 oder SEQ ID NO: 72 umfasst.

**6.** Polynukleotid oder Genkonstrukt, welches ein Konjugat gemäß irgendeinem der Ansprüche 3 bis 5 codiert.

**7.** Zelle, die ein Polynukleotid oder ein Genkonstrukt gemäß Anspruch 6 enthält.

**8.** Zusammensetzung, die zusammen oder separat umfasst:

(i) ein Konjugat gemäß irgendeinem der Ansprüche 1 bis 5, ein Polynukleotid oder Genkonstrukt gemäß Anspruch 6 oder eine Wirtszelle gemäß Anspruch 7 und
(ii) einen TLR-Liganden.

**9.** Zusammensetzung, die zusammen oder separat umfasst:

(i) ein Konjugat gemäß irgendeinem der Ansprüche 1 bis 5, ein Polynukleotid oder Genkonstrukt gemäß Anspruch 6 oder eine Wirtszelle gemäß Anspruch 7,
(ii) einen TLR-Liganden und
(iii) ein chemotherapeutisches Mittel.

**10.** Zusammensetzung gemäß den Ansprüchen 8 oder 9, wobei der TLR-Ligand aus der Gruppe ausgewählt ist, bestehend aus einem TLR3-Liganden, einem TLR9-Liganden und einer Kombination von beiden.

**11.** Zusammensetzung gemäß Anspruch 10, wobei der TLR3-Ligand poly(I:C) ist, wobei der TLR9-Ligand ein Oligonukleotid ist, welches wenigstens ein CpG-Motiv umfasst, oder wobei das chemotherapeutische Mittel (iii) Cyclophosphamid ist.

**12.** Pharmazeutische Zusammensetzung, welche ein Konjugat gemäß irgendeinem der Ansprüche 1 bis 5, ein Polynukleotid oder Genkonstrukt gemäß Anspruch 6, eine Zelle gemäß Anspruch 7 oder eine Zusammensetzung gemäß irgendeinem der Ansprüche 8 bis 11 und wenigstens ein/en pharmakologisch annehmbare(s/n) Träger oder Adjuvans umfasst.

**13.** Konjugat gemäß irgendeinem der Ansprüche 1 bis 5, Polynukleotid oder Genkonstrukt gemäß Anspruch 6, Zelle gemäß Anspruch 7 oder Zusammensetzung gemäß irgendeinem der Ansprüche 8 bis 11 zur Verwendung in der Medizin.

**14.** Verwendung eines Konjugats gemäß irgendeinem der Ansprüche 1 bis 5, eines Polynukleotids oder Genkonstrukts gemäß Anspruch 6, einer Zelle gemäß Anspruch 7 oder einer Zusammensetzung gemäß irgendeinem der Ansprüche 8 bis 11 zur Herstellung eines Impfstoffs.

**15.** Konjugat gemäß irgendeinem der Ansprüche 1 bis 5, Polynukleotid oder Genkonstrukt gemäß Anspruch 6, Zelle gemäß Anspruch 7 oder Zusammensetzung gemäß irgendeinem der Ansprüche 8 bis 11 zur Verwendung bei der Prävention oder Behandlung einer Infektion, verursacht durch das humane Papillomvirus, und/oder von mit HPV-Infektionen assoziiertem Gebärmutterhalskrebs.

**16.** *In vitro*-Verfahren zum Erhalten reifer dendritischer Zellen, die wenigstens ein HPV E7-Antigen präsentieren, umfassend:

i) Inkontaktbringen dendritischer Zellen mit einem Konjugat gemäß irgendeinem der Ansprüche 1 bis 5, einem Polynukleotid oder Genkonstrukt gemäß Anspruch 6, einer Zelle gemäß Anspruch 7 oder einer Zusammensetzung gemäß irgendeinem der Ansprüche 8 bis 11 in Bedingungen, die geeignet sind, damit die Reifung der dendritischen Zellen stattfinden kann, und
ii) Gewinnen der reifen dendritischen Zellen.

## Revendications

**1.** Conjugué comprenant :

i) la région EDA de la fibronectine ou l'un de ses variants fonctionnellement équivalents présentant un degré d'identité par rapport à la région EDA de la fibronectine supérieur à au moins 70 % et
ii) au moins un peptide antigénique dérivé de E7 du PVH, dans lequel l'au moins un peptide antigénique dérivé de E7 du PVH est un fragment de la protéine E7 du PVH qui est capable de stimuler le système immunitaire d'un mammifère de telle façon qu'une réponse immunitaire contre ladite protéine capable d'inhiber la croissance de tumeurs provoquées par l'expression de E7 ou d'inhiber la prolifération du PVH est produite,

dans lequel les composants (i) et (ii) sont couplés de façon covalente et dans lequel le conjugué favorise une réponse cytotoxique envers le peptide ou les peptides antigéniques.

**2.** Conjugué selon la revendication 1, dans lequel le composant (ii) comprend un peptide antigénique contenant les acides aminés 1 à 29 de SEQ ID NO : 51, un peptide antigénique contenant les acides aminés 43 à 98 de SEQ ID

NO : 52 ou les deux.

**3.** Conjugué selon l'une quelconque des revendications 1 ou 2, dans lequel le composant (ii) forme une chaîne polypeptidique unique avec le composant (i).

**4.** Conjugué selon l'une quelconque des revendications 1 à 3, dans lequel la région EDA est flanquée de deux peptides antigéniques E7 du PVH.

**5.** Conjugué selon la revendication 4, comprenant la séquence SEQ ID NO : 53 ou SEQ ID NO : 72.

**6.** Polynucléotide ou construction génique codant pour un conjugué selon l'une quelconque des revendications 3 à 5.

**7.** Cellule contenant un polynucléotide ou une construction génique selon la revendication 6.

**8.** Composition comprenant, conjointement ou séparément :

(i) un conjugué selon l'une quelconque des revendications 1 à 5, un polynucléotide ou une construction génique selon la revendication 6, ou une cellule hôte selon la revendication 7 et
(ii) un ligand de TLR.

**9.** Composition comprenant, conjointement ou séparément :

(i) un conjugué selon l'une quelconque des revendications 1 à 5, un polynucléotide ou une construction génique selon la revendication 6, ou une cellule hôte selon la revendication 7,
(ii) un ligand de TLR et
(iii) un agent chimiothérapeutique.

**10.** Composition selon les revendications 8 ou 9, dans laquelle le ligand de TLR est choisi dans le groupe constitué d'un ligand du TLR3, un ligand du TLR9 et une combinaison des deux.

**11.** Composition selon la revendication 10, dans laquelle le ligand du TLR3 est un poly(I:C), dans laquelle le ligand du TLR9 est un oligonucléotide comprenant au moins un motif CpG ou dans laquelle l'agent chimiothérapeutique (iii) est le cyclophosphamide.

**12.** Composition pharmaceutique comprenant un conjugué selon l'une quelconque des revendications 1 à 5, un polynucléotide ou une construction génique selon la revendication 6, une cellule selon la revendication 7 ou une composition selon l'une quelconque des revendications 8 à 11 et au moins un support ou un adjuvant acceptable d'un point de vue pharmacologique.

**13.** Conjugué selon l'une quelconque des revendications 1 à 5, polynucléotide ou construction génique selon la revendication 6, cellule selon la revendication 7 ou composition selon l'une quelconque des revendications 8 à 11 pour son utilisation en médecine.

**14.** Utilisation d'un conjugué selon l'une quelconque des revendications 1 à 5, d'un polynucléotide ou d'une construction génique selon la revendication 6, d'une cellule selon la revendication 7 ou d'une composition selon l'une quelconque des revendications 8 à 11 pour la fabrication d'un vaccin.

**15.** Conjugué selon l'une quelconque des revendications 1 à 5, polynucléotide ou construction génique selon la revendication 6, cellule selon la revendication 7 ou composition selon l'une quelconque des revendications 8 à 11 pour une utilisation dans la prévention ou le traitement d'une infection provoquée par le papillomavirus humain et/ou du cancer du col de l'utérus associé à des infections par le PVH.

**16.** Procédé *in vitro* pour l'obtention de cellules dendritiques matures présentant au moins un antigène E7 du PVH, comprenant :

i) la mise en contact de cellules dendritiques avec un conjugué selon l'une quelconque des revendications 1 à 5, un polynucléotide ou une construction génique selon la revendication 6, une cellule selon la revendication 7 ou une composition selon l'une quelconque des revendications 8 à 11 dans des conditions appropriées pour

que la maturation des cellules dendritiques prenne place et ii) la récupération des cellules dendritiques matures.

A

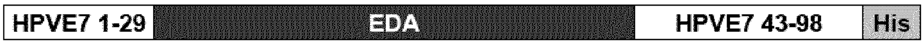


B

MHGDTPTLHEYMLDLQPETTDLYCYEQLNHMNIDR
PKGLAFTDVDVDSIKIAWESPQGQVSRYRVTYSSPE
DGIRELFPAPDGEDDTAELQGLRPGSEYTVSVVAL
HDDMESQPLIGIQSTAAAGQAEPDRAHYNIVTFCCK
CDSTLRLCVQSTHVDIRTLEDLLMGTLGIVCPICSQK
PAAALEHHHHHH

C

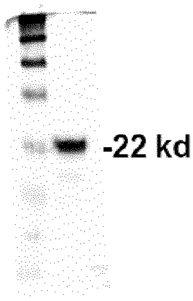


Figure 1

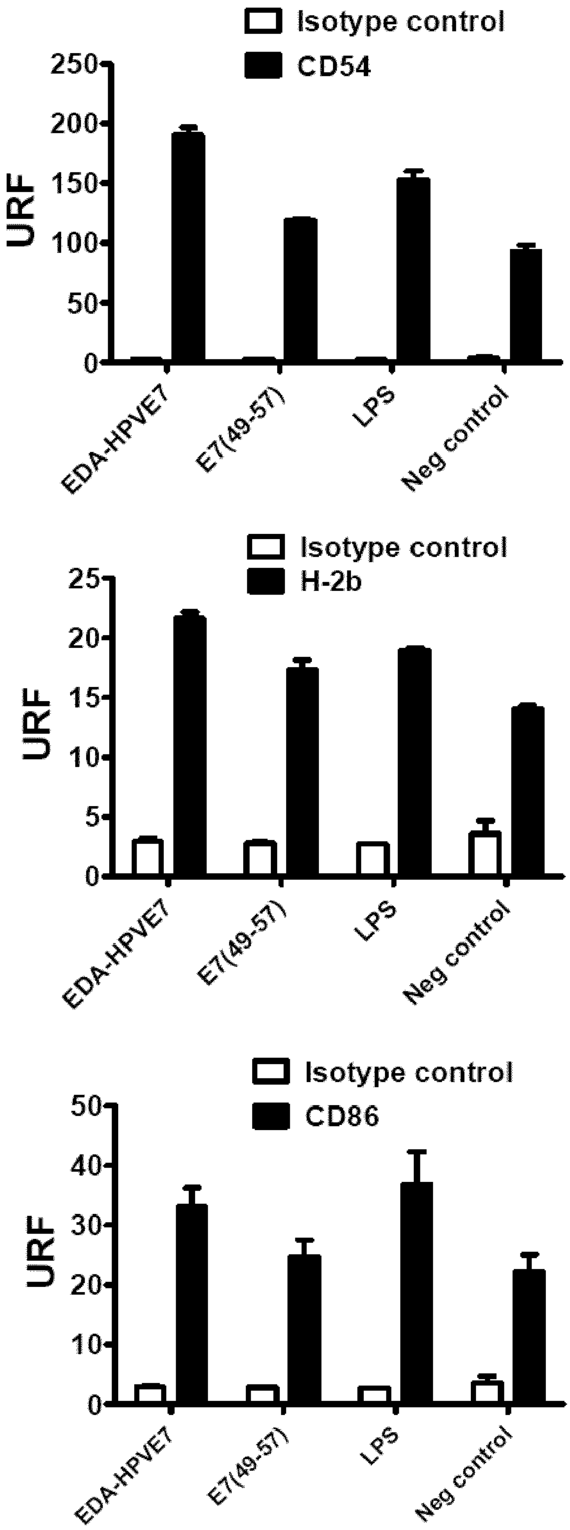
Isotype control
CD54
250
200
150
100
50
0
URF
EDA-HPVE7
E7(49-57)
LPS
Neg control
Isotype control
H-2b
25
20
15
10
5
0
URF
EDA-HPVE7
E7(49-57)
LPS
Neg control
Isotype control
CD86
50
40
30
20
10
0
URF
EDA-HPVE7
E7(49-57)
LPS
Neg control

Figure 2

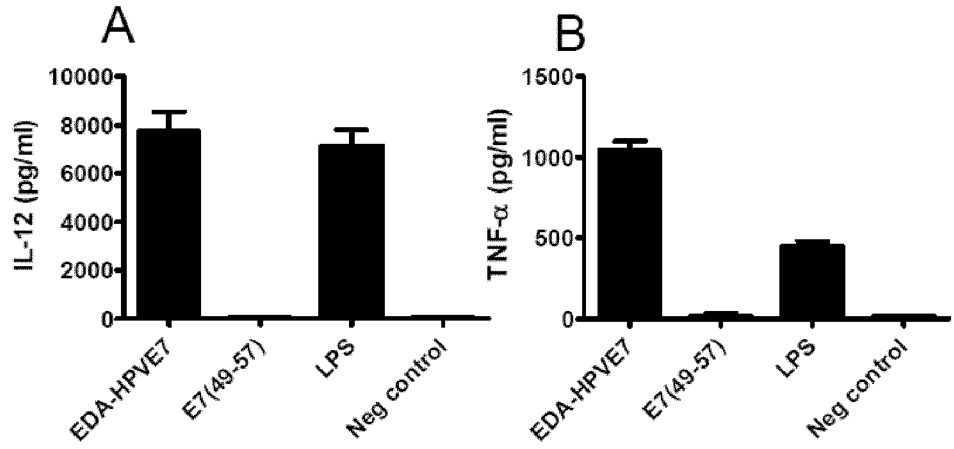
A
IL-12 (pg/ml)
10000
8000
6000
4000
2000
0
EDA-HPVE7
E7(49-57)
LPS
Neg control
B
TNF-α (pg/ml)
1500
1000
500
0
EDA-HPVE7
E7(49-57)
LPS
Neg control

Figure 3

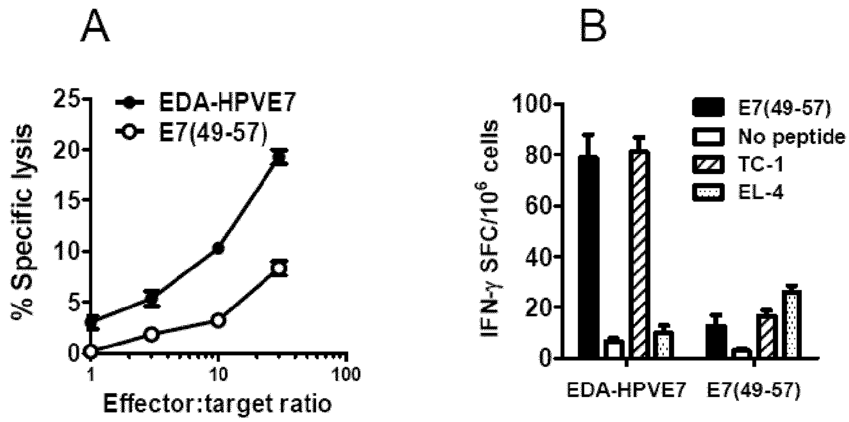
A
B
25
20
15
10
5
0
1
10
100
% Specific lysis
Effector:target ratio
EDA-HPVE7
E7(49-57)
100
80
60
40
20
0
IFN-γ SFC/10^6 cells
E7(49-57)
No peptide
TC-1
EL-4
EDA-HPVE7
E7(49-57)

Figure 4

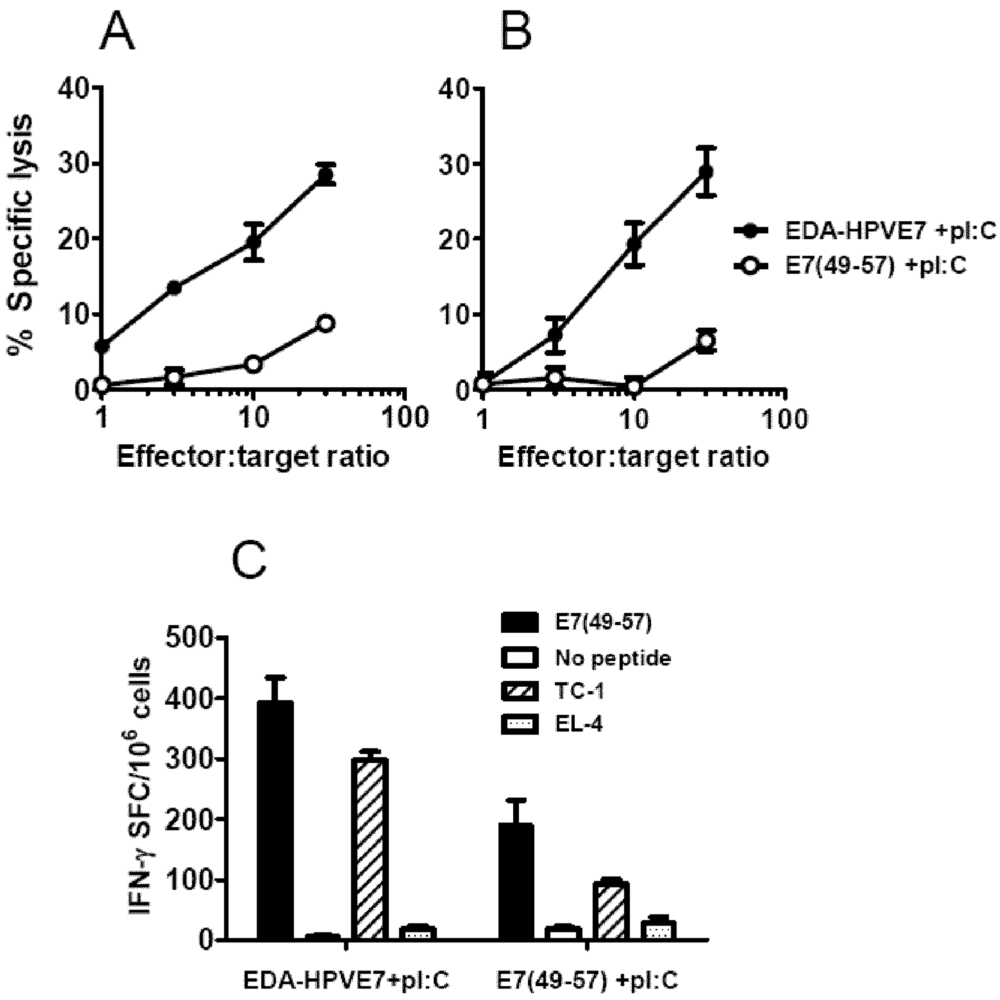
A
B
% Specific lysis
40
30
20
10
0
1
10
100
Effector:target ratio
EDA-HPVE7 +pI:C
E7(49-57) +pI:C
C
IFN-γ SFC/10^6 cells
500
400
300
200
100
E7(49-57)
No peptide
TC-1
EL-4
EDA-HPVE7+pI:C
E7(49-57) +pI:C

Figure 5

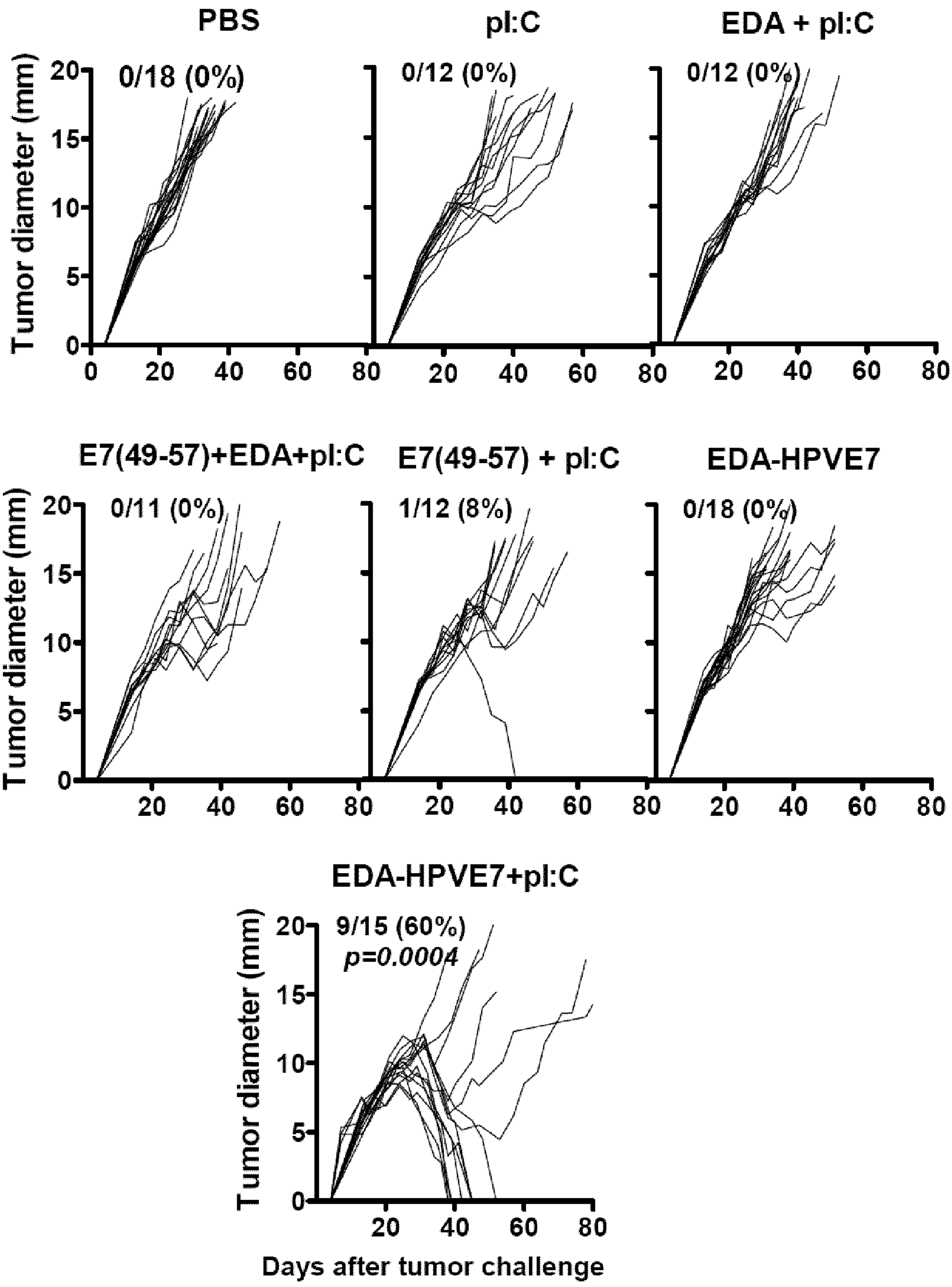
PBS
pI:C
EDA + pI:C
0/18 (0%)
0/12 (0%)
0/12 (0%)
E7(49-57)+EDA+pI:C
E7(49-57) + pI:C
EDA-HPVE7
0/11 (0%)
1/12 (8%)
0/18 (0%)
EDA-HPVE7+pI:C
9/15 (60%)
p=0.0004
Tumor diameter (mm)
0
5
10
15
20
0
20
40
60
80
Days after tumor challenge

Figure 6A

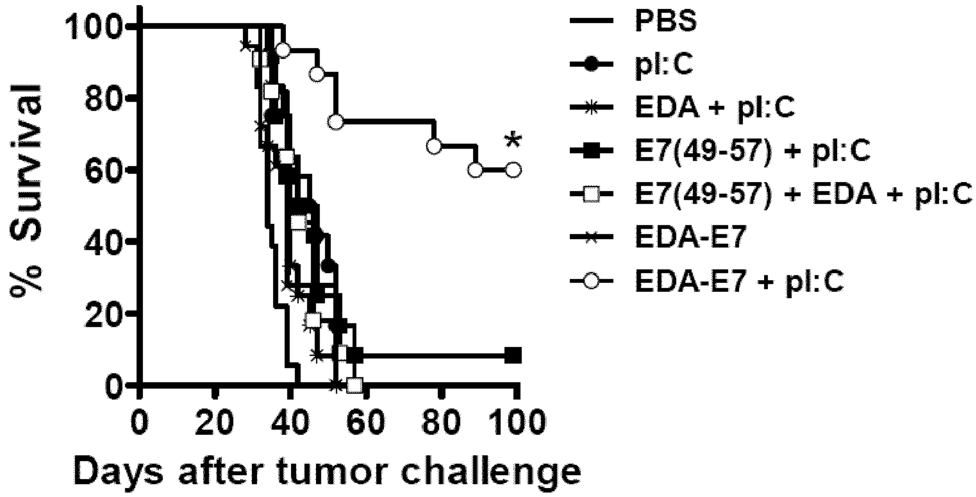
100
80
60
40
20
0
% Survival
0
20
40
60
80
100
Days after tumor challenge
*
PBS
pI:C
EDA + pI:C
E7(49-57) + pI:C
E7(49-57) + EDA + pI:C
EDA-E7
EDA-E7 + pI:C

Figure 6B

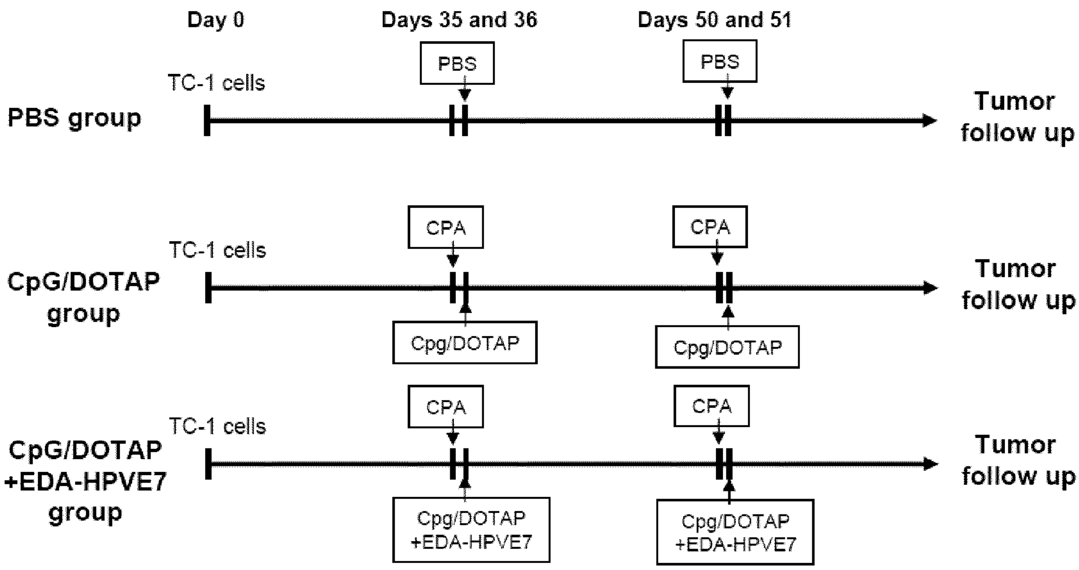
Day 0
Days 35 and 36
Days 50 and 51
TC-1 cells
PBS group
PBS
PBS
Tumor follow up
TC-1 cells
CpG/DOTAP group
CPA
CPA
Cpg/DOTAP
Cpg/DOTAP
Tumor follow up
TC-1 cells
CpG/DOTAP +EDA-HPVE7 group
CPA
CPA
Cpg/DOTAP +EDA-HPVE7
Cpg/DOTAP +EDA-HPVE7
Tumor follow up

Figure 7A

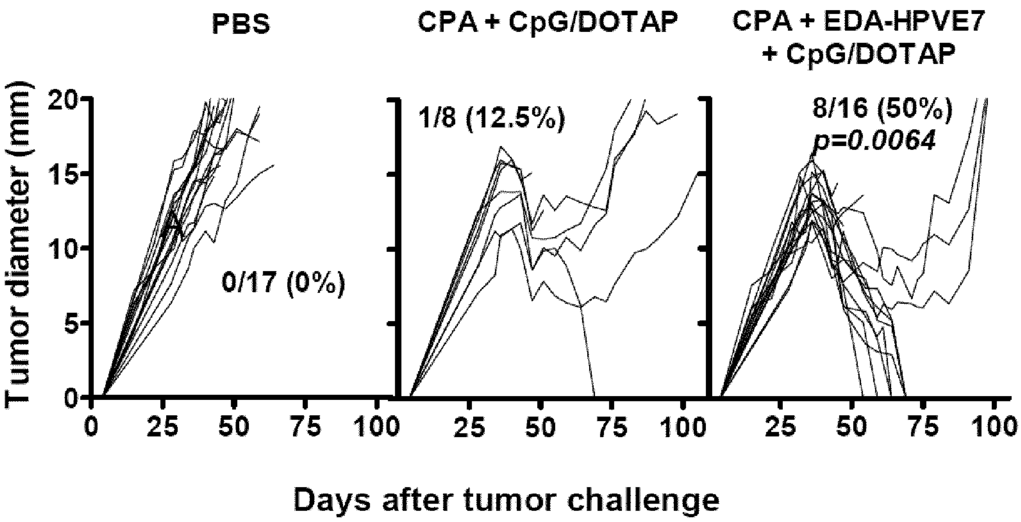
PBS
CPA + CpG/DOTAP
CPA + EDA-HPVE7
+ CpG/DOTAP
Tumor diameter (mm)
20
15
10
5
0
0/17 (0%)
1/8 (12.5%)
8/16 (50%)
p=0.0064
0
25
50
75
100
Days after tumor challenge

Figure 7B

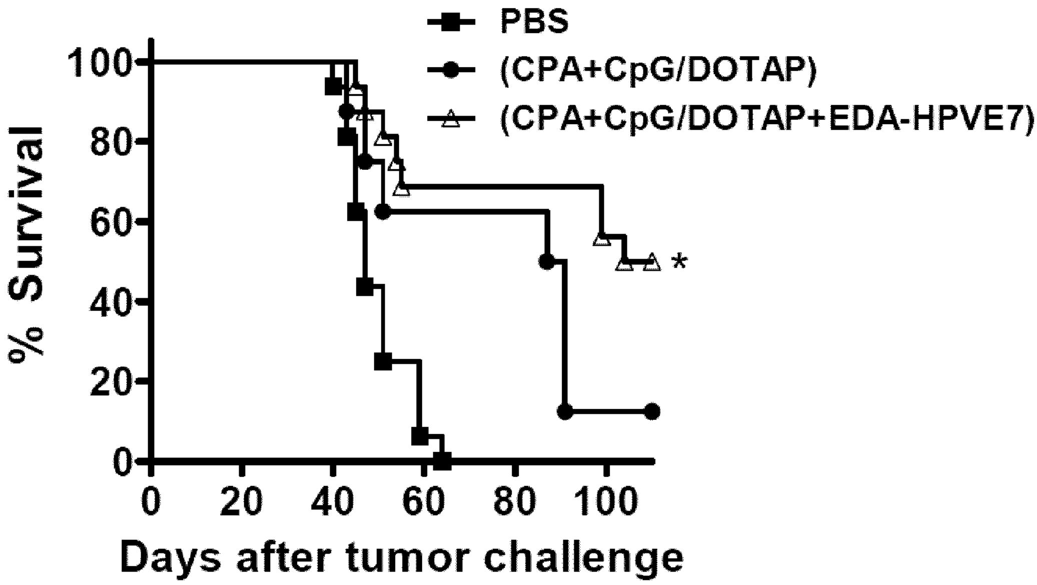
PBS
(CPA+CpG/DOTAP)
(CPA+CpG/DOTAP+EDA-HPVE7)
100
80
60
40
20
0
% Survival
*
0
20
40
60
80
100
Days after tumor challenge

Figure 7C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4777239 A
- EP 0796273 A
- EP 1334177 B1
- US 20050255042 A
- WO 04054622 A
- WO 06107617 A
- WO 07046893 A
- WO 07112193 A
- US 6809186 B
- WO 2006135436 A
- US 5190929 A
- WO 07069090 A
- WO 9630030 A
- WO 2002048167 A
- WO 06134190 A


### Non-patent literature cited in the description


- **SEEDORF, K. et al.** *Virology,* 1985, vol. 145, 181-185
- **KLUG, A. J.** *Mol. Biol.,* 1965, vol. 11, 403-423
- **STOPPLER et al.** *Intervirology,* 1994, vol. 37, 168-179
- **SEEDORF et al.** *Virology,* 1985, vol. 145, 181-185
- **PRÉVILLE et al.** *Cancer Res.,* 2005, vol. 65, 641-649
- **BERRAONDO P et al.** *Cancer research,* 2007, vol. 67, 8847-8855
- **MURO A. F. et al.** *J. Cell. Biol.,* 2003, vol. 162, 149-160
- **ALTSCHUL S.F. et al.** Basic local alignment search tool. *J Mol Biol.,* 05 October 1990, vol. 215 (3), 403-10
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3, 547-553
- **HOPP et al.** *BioTechnology,* 1988, vol. 6, 1204-1210
- **MARTIN et al.** *Science,* 1993, vol. 255, 192-194
- **MULLER, K.M. ; ARNDT, K.M. ; ALBER, T.** *Meth. Enzimology,* 2000, vol. 328, 261-281
- **NIELSEN, B.B. et al.** *FEBS Lett.,* 1997, vol. 412, 388-396
- **PACK P. ; PLUCKTHUN, A.** *Biochemistry,* 1992, vol. 31, 1579-1584
- **MIYATAKE et al.** *J. Virol,* 1997, vol. 71, 5124-32
- **SANDIG et al.** *Gene Ther.,* 1996, vol. 3, 1002-9
- **ARBUTHNOT et al.** *Hum.GeneTher.,* 1996, vol. 7, 1503-14
- **WANG, L. et al.** *Proc.Natl.Acad.Sci.USA,* 1997, vol. 94, 11563-11566
- **MASOOD, R. et al.** *Int J Mol Med,* 2001, vol. 8, 335-343
- **SOMIA, N.V. et al.** *Proc Acad Sci USA,* 1995, vol. 92, 7570-7574
- **SUDOWE, S. et al.** *J Allergy Clin Immunol.,* 2006, vol. 117, 196-203
- *Gene Ther.,* 2001, vol. 8, 1729-1737
- **SAMBROOK et al.** Molecular cloning, to Laboratory Manual. Cold Spring Harbor Laboratory Press, 2001, vol. 1-3
- **DU X et al.** *Eur.Cytokine Netw.,* 2000, vol. 11, 362-71
- **CHUANG TH et al.** *Eur. Cytokine Netw.,* 2000, vol. 11, 372-378
- **TABETA K et al.** *Proc. Natl. Acad. Sci. U.S.A.,* 2004, vol. 101, 3516-3521
- **CHEN X.Z. et al.** *Arch Dermatol Res.,* 04 July 2009
- Cyclophosphamide. Merck Index. 429-430
- **E.W. MARTIN.** Remington's Pharmaceutical Sciences. 1995
- **C. FAULÍ I TRILLO.** Tratado de Farmacia Galénica. 1993
- Remington's Pharmaceutical Sciences. Williams & Wilkins PA, 2000
- **TEMPLETON.** *DNA Cell Biol.,* 2002, vol. 21, 857-867
- **LINDGREN, A. et al.** *rends Pharmacol. Sci,* 2000, vol. 21, 99-103
- **SCHWARZE, S.R. et al.** *Trends Pharmacol. Sci.,* 2000, vol. 21, 45-48
- **LUNDBERG, M et al.** *Mol Therapy,* 2003, vol. 8, 143-150

- **SNYDER, E.L. ; DOWDY, S.F.** *Pharm. Res.,* 2004, vol. 21, 389-393
- **GHIRINGHELLI et al.** *Cancer Immunol. Immunother.,* 2007, vol. 56, 641-8
- **KAISHO ; AKIRA.** *Biochimica et Biophysica Acta,* 2002, vol. 1589, 1-13
- **PAGLIA P. et al.** *Journal of Experimental Medicine,* 1993, vol. 178, 1893-1901